# EUROPEAN PATENT SPECIFICATION

(11) **EP 2 270 171 B1**
(45) Date of publication and mention of the grant of the patent: **04.09.2013**
(21) Application number: 10177857.9
(22) Date of filing: 02.10.2001
(51) Int. Cl.: C12N 15/31, C12N 15/63, C07K 14/285, C07K 19/00, A61K 39/102, G01N 33/569

(54) **Haemophilus influenzae antigens and corresponding DNA fragments**
Haemophilus influenzae-Antigene und entsprechende DNA-Fragmente
Antigènes de Haemophilus influenzae et fragments d'ADN correspondants

(30) Priority: 02.10.2000 US 236712 P
(43) Date of publication of application: 05.01.2011
(62) Divisional of application: 09159896.1
(73) Proprietor: ID Biomedical Corporation of Quebec, Laval, QC H7V 3S8 (CA)
(72) Inventor: Hamel, Josée, Sillery, Québec G1T 1N6 (CA); Couture, France, St-Ferréol-les-neiges Québec G0A 3RO (CA); Brodeur, Bernard R, Sillery, Québec G1T 1N6 (CA); Martin, Denis, St-Augustin-de-Desmaures Québec G3A 1E9 (CA); Ouellet, Catherine, St-Jean-Chrysostome Québec G6Z 2Z8 (CA); Tremblay, Mireille, Québec, Québec G2B 5E1 (CA); Charbonneau, Annie, Québec, Québec G1S 1Z5 (CA); Vayssier, Catherine, Sillery, Québec G1T 1H8 (CA)
(74) Representative: Lubienski, Michael John

(56) References cited:
- WO-A-96/33276
- DATABASE UniProt [Online] EBI; 1 November 1995 (1995-11-01), "Spermidine/putrescine-binding periplasmic protein 1 precursor (SPBP) of H. influenzae" XP002532786 Database accession no. P45168
- ZAGURSKY ROBERT J ET AL: "Identification of a Haemophilus influenzae 5'-nucleotidase protein: Cloning of the nucA gene and immunogenicity and characterization of the NucA protein." INFECTION AND IMMUNITY, vol. 68, no. 5, May 2000 (2000-05), pages 2525-2534, XP002214523 ISSN: 0019-9567
- KARALUS RICHARD J ET AL: "Purification and characterization of outer membrane protein P6, a vaccine antigen of non-typeable Haemophilus influenzae." FEMS IMMUNOLOGY AND MEDICAL MICROBIOLOGY, vol. 26, no. 2, November 1999 (1999-11), pages 159-166, XP002214524 ISSN: 0928-8244
- LOOSMORE S M ET AL: "OUTER MEMBRANE PROTEIN D15 IS CONSERVED AMONG HAEMOPHILUS INFLUENZAE SPECIES AND MAY REPRESENT A UNIVERSAL PROTECTIVE ANTIGEN AGAINST INVASIVE DISEASE" INFECTION AND IMMUNITY, AMERICAN SOCIETY FOR MICROBIOLOGY. WASHINGTON, US, vol. 9, no. 65, September 1997 (1997-09), pages 3701-3707, XP002951795 ISSN: 0019-9567
- BARENKAMP S J ET AL: "CLONING, EXPRESSION, AND DNA SEQUENCE ANALYSIS OF GENES ENCODING NONTYPEABLE HAEMOPHILUS INFLUENZAE HIGH-MOLECULAR-WEIGHT SURFACE-EXPOSED PROTEINS RELATED TO FILAMENTOUS HEMAGGLUTININ OF BORDETELLA PERTUSSIS" INFECTION AND IMMUNITY, AMERICAN SOCIETY FOR MICROBIOLOGY. WASHINGTON, US, vol. 60, no. 4, 1 April 1992 (1992-04-01), pages 1302-1313, XP000578343 ISSN: 0019-9567
- MITCHELL J L ET AL: "Neutrophil opsonophagocytosis of particles coated with surface antigens of nontypeable Haemophilus influenzae." ABSTRACTS OF THE GENERAL MEETING OF THE AMERICAN SOCIETY FOR, vol. 95, 1995, page 281, XP002214525 95th General Meeting of the American Society for Microbiology;Washington, D.C., USA; May 21-25, 1995, 1995 ISSN: 1060-2011
- DATABASE UniProt [Online] EBI; 1 November 1995 (1995-11-01), "Haemophilus influenza uncharacterized protein HI0178", XP002663408, Database accession no. P43961

<110> SHIRE BIOCHEM INC.
   HAMEL, Josee
   COUTURE, France
   BRODEUR, Bernard R.
   MARTIN, Denis
<120> HAEMOPHILUS INFLUENZAE ANTIGENS AND CORRESPONDING DNA FRAGMENTS
<130> 484112.440EP1
<150> PCT/CA01/01402
   <151> 2001-10-02
<150> 60/236,712
   <151> 2000-10-02
<160> 51
<170> PatentIn version 3.1
<210> 1
   <211> 1140
   <212> DNA
   <213> Haemophilus influenzae
<400> 1
   atgcctgtca ttcggcaggt ggttttttac gactctttaa caggagaaca aacaaaaatg      60
   aaaaaatttg caggtttaat tactgccagc ttcgtagctg caactttaac cgcttgcaac     120
   gataaagatg ctaagcaaga aactgcaaaa gctacggctg ctgctaatga cactgtgtat     180
   ctctacactt ggacagaata tgtaccagat ggtcttttag atgagtttac taaagaaact     240
   ggcatcaaag ttatcgtttc aagcctagaa tcaaacgaaa caatgtacgc aaaactcaaa     300
   actcaaggcg agtctggtgg ctatgatgtt atcgcacctt ctaactactt cgtttctaaa     360
   atggcacgcg aaggaatgtt aaaagaactt gatcacagca aattacctgt tcttaaagaa     420
   ttagatcctg attggctcaa taaaccttat gataaaggta acaaatactc tcttccgcaa     480
   cttttaggtg cgccgggtat cgcattcaat accaacactt acaaaggcga acaattcact     540
   tcttgggcag acttatggaa acctgaattt gcaaacaaag ttcagttatt agacgatgcg     600
   cgcgaagtat tcaacatcgc tttattgaaa attggtcaag atccaaatac tcaagatcca     660
   gccattatca aacaagccta tgaagaatta ttgaaattac gtccaaatgt actttctttc     720
   aattccgata acccagctaa ctcgttcatc tcaggcgaag tggaagtggg tcaattatgg     780
   aatggttcgg tacgtattgc taaaaaagaa aaagcacctt taaatatggt attcccaaaa     840
   gagggtcctg tactttgggt tgatacactt gcaattcctg taacagctaa aaatcctgaa     900
   ggcgcacaca agctgattaa ctacatgtta gggaaaaaaa cagctgaaaa attaacctta     960
   gctatcggtt acccaacctc aaatattgaa gcgaaaaaag cattaccaaa agaaatcact    1020
   gaagatccag cgatttatcc gtcagctgat atattaaaaa atagtcactg gcaagatgac    1080
   gttggcgatg caattcaatt ctatgaacaa tattatcaag aattaaaagc agcaaaataa    1140
<210> 2
   <211> 379
   <212> PRT
   <213> Haemophilus influenzae
<220>
   <221> SIGNAL
   <222> (1)..(33)
<400> 2
   Met Pro Val Ile Arg Gln Val Val Phe Tyr Asp Ser Leu Thr Gly Glu
   1               5                   10                  15
   Gln Thr Lys Met Lys Lys Phe Ala Gly Leu Ile Thr Ala Ser Phe Val
               20                  25                  30
   Ala Ala Thr Leu Thr Ala Cys Asn Asp Lys Asp Ala Lys Gln Glu Thr
           35                  40                  45
   Ala Lys Ala Thr Ala Ala Ala Asn Asp Thr Val Tyr Leu Tyr Thr Trp
       50                  55                  60
   Thr Glu Tyr Val Pro Asp Gly Leu Leu Asp Glu Phe Thr Lys Glu Thr
   65                  70                  75                  80
   Gly Ile Lys Val Ile Val Ser Ser Leu Glu Ser Asn Glu Thr Met Tyr
                   85                  90                  95
   Ala Lys Leu Lys Thr Gln Gly Glu Ser Gly Gly Tyr Asp Val Ile Ala
               100                 105                 110
   Pro Ser Asn Tyr Phe Val Ser Lys Met Ala Arg Glu Gly Met Leu Lys
           115                 120                 125
   Glu Leu Asp His Ser Lys Leu Pro Val Leu Lys Glu Leu Asp Pro Asp
       130                 135                 140
   Trp Leu Asn Lys Pro Tyr Asp Lys Gly Asn Lys Tyr Ser Leu Pro Gln
   145                 150                 155                 160
   Leu Leu Gly Ala Pro Gly Ile Ala Phe Asn Thr Asn Thr Tyr Lys Gly
                       165                 170                 175
   Glu Gln Phe Thr Ser Trp Ala Asp Leu Trp Lys Pro Glu Phe Ala Asn
                   180                 185                 190
   Lys Val Gln Leu Leu Asp Asp Ala Arg Glu Val Phe Asn Ile Ala Leu
               195                 200                 205
   Leu Lys Ile Gly Gln Asp Pro Asn Thr Gln Asp Pro Ala Ile Ile Lys
       210                 215                 220
   Gln Ala Tyr Glu Glu Leu Leu Lys Leu Arg Pro Asn Val Leu Ser Phe
   225                 230                 235                 240
   Asn Ser Asp Asn Pro Ala Asn Ser Phe Ile Ser Gly Glu Val Glu Val
                   245                 250                 255
   Gly Gln Leu Trp Asn Gly Ser Val Arg Ile Ala Lys Lys Glu Lys Ala
               260                 265                 270
   Pro Leu Asn Met Val Phe Pro Lys Glu Gly Pro Val Leu Trp Val Asp
           275                 280                 285
   Thr Leu Ala Ile Pro Val Thr Ala Lys Asn Pro Glu Gly Ala His Lys
       290                 295                 300
   Leu Ile Asn Tyr Met Leu Gly Lys Lys Thr Ala Glu Lys Leu Thr Leu
   305                 310                 315                 320
   Ala Ile Gly Tyr Pro Thr Ser Asn Ile Glu Ala Lys Lys Ala Leu Pro
                   325                 330                 335
   Lys Glu Ile Thr Glu Asp Pro Ala Ile Tyr Pro Ser Ala Asp Ile Leu
               340                 345                 350
   Lys Asn Ser His Trp Gln Asp Asp Val Gly Asp Ala Ile Gln Phe Tyr
           355                 360                 365
   Glu Gln Tyr Tyr Gln Glu Leu Lys Ala Ala Lys
       370                 375
<210> 3
   <211> 1008
   <212> DNA
   <213> Haemophilus influenzae
<400> 3
   atgaaaaaac ttttaaaaat tagtgccgtt tctgccgcac ttttaagtgc gccaatgatg      60
   gcgaatgccg atgtattagc atcagtaaaa cctttaggct ttattgtttc atctattgca     120
   gatggcgtaa ctggtacaca agtccttgtt cctgctggcg cctctccgca tgattacaat     180
   ttgaaattat ctgatattca aaaagtaaaa tctgcagatt tagttgtatg gattggtgaa     240
   gacattgatt cattcttaga caaaccaatt agccaaattg aacgtaaaaa agtgattacc     300
   attgccgatc ttgcggatgt aaaaccttta ttaagtaaag ctcaccacga gcatttccat     360
   gaagatggcg atcatgatca tgaccataag cacgaacaca aacatgatca taaacatgac     420
   catgaccatg atcataaaca cgagcataaa cacgatcacg aacatcatga tcacgatcat     480
   cacgagggtt taacaacaaa ctggcacgtt tggtattctc cagctatcag caaaattgtt     540
   gcacaaaaag tagcggataa attaactgca caattcccag ataaaaaagc gttaattgca     600
   caaaatcttt cagattttaa ccgcactttg gcagaacaaa gtgaaaaaat tacggcacaa     660
   cttgcaaatg ttaaagataa aggtttctac gttttccacg atgcttatgg ttatttcaac     720
   gatgcttatg ggttaaaaca aacaggttac tttaccatca atccattagt agcaccgggt     780
   gcaaaaactt tagcgcacat taaagaagaa attgatgaac ataaagtaaa ttgcttattc     840
   gcagagcctc aatttacgcc aaaagtgatt gagtctttag cgaaaaatac taaagtcaat     900
   gtaggacagc tcgacccaat tggcgataaa gttactttag gtaaaaattc ttatgcaaca     960
   ttcttgcaat ctactgcaga tagctacata gaatgtttag ctaaataa                  1008
<210> 4
   <211> 335
   <212> PRT
   <213> Haemophilus influenzae
<220>
   <221> SIGNAL
   <222> (1)..(21)
<400> 4
   Met Lys Lys Leu Leu Lys Ile Ser Ala Val Ser Ala Ala Leu Leu Ser
   1               5                   10                  15
   Ala Pro Met Met Ala Asn Ala Asp Val Leu Ala Ser Val Lys Pro Leu
               20                  25                  30
   Gly Phe Ile Val Ser Ser Ile Ala Asp Gly Val Thr Gly Thr Gln Val
           35                  40                  45
   Leu Val Pro Ala Gly Ala Ser Pro His Asp Tyr Asn Leu Lys Leu Ser
       50                  55                  60
   Asp Ile Gln Lys Val Lys Ser Ala Asp Leu Val Val Trp Ile Gly Glu
   65                  70                  75                  80
   Asp Ile Asp Ser Phe Leu Asp Lys Pro Ile Ser Gln Ile Glu Arg Lys
                   85                  90                  95
   Lys Val Ile Thr Ile Ala Asp Leu Ala Asp Val Lys Pro Leu Leu Ser
               100                 105                 110
   Lys Ala His His Glu His Phe His Glu Asp Gly Asp His Asp His Asp
           115                 120                 125
   His Lys His Glu His Lys His Asp His Lys His Asp His Asp His Asp
       130                 135                 140
   His Lys His Glu His Lys His Asp His Glu His His Asp His Asp His
   145                 150                 155                 160
   His Glu Gly Leu Thr Thr Asn Trp His Val Trp Tyr Ser Pro Ala Ile
                   165                 170                 175
   Ser Lys Ile Val Ala Gln Lys Val Ala Asp Lys Leu Thr Ala Gln Phe
               180                 185                 190
   Pro Asp Lys Lys Ala Leu Ile Ala Gln Asn Leu Ser Asp Phe Asn Arg
           195                 200                 205
   Thr Leu Ala Glu Gln Ser Glu Lys Ile Thr Ala Gln Leu Ala Asn Val
       210                 215                 220
   Lys Asp Lys Gly Phe Tyr Val Phe His Asp Ala Tyr Gly Tyr Phe Asn
   225                 230                 235                 240
   Asp Ala Tyr Gly Leu Lys Gln Thr Gly Tyr Phe Thr Ile Asn Pro Leu
                   245                 250                 255
   Val Ala Pro Gly Ala Lys Thr Leu Ala His Ile Lys Glu Glu Ile Asp
               260                 265                 270
   Glu His Lys Val Asn Cys Leu Phe Ala Glu Pro Gln Phe Thr Pro Lys
           275                 280                 285
   Val Ile Glu Ser Leu Ala Lys Asn Thr Lys Val Asn Val Gly Gln Leu
       290                 295                 300
   Asp Pro Ile Gly Asp Lys Val Thr Leu Gly Lys Asn Ser Tyr Ala Thr
   305                 310                 315                 320
   Phe Leu Gln Ser Thr Ala Asp Ser Tyr Ile Glu Cys Leu Ala Lys
                   325                 330                 335
<210> 5
   <211> 1650
   <212> DNA
   <213> Haemophilus influenzae
<400> 5
   atgcttatga aactaaaagc aacattaact ctcgccgctg caactcttgt tttggcagct     60
   tgtgatcaat ctagctcggc aaataaatct accgctcaaa ctgaagcaaa atcctcgtca    120
   aataatactt tcgtttattg cacagcgaaa gctccattgg gatttagccc tgcgttaata    180
   attgaaggta cttcttataa tgcaagctca caacaagtgt ataaccgctt agttgaattt    240
   aaaaaaggct caacggatat tgaaccagct ttagctgaaa gctgggaaat tagcgatgat    300
   ggcttaagtt atacttttca tttgagaaaa ggggttaaat tccacacaac aaaagaattt    360
   accccgacac gtgattttaa tgcagatgat gttgtatttt cattccaacg tcagttagat    420
   ccaaatcatc catatcacaa tgtatctaag gggacttatc catattttaa agcaatgaaa    480
   ttccctgaat tgttgaaatc agtggagaaa gtagacgata acacaattcg tattacctta    540
   aacaagacag atgcgacttt cttggctagt cttggtatgg attttatttc tatttattct    600
   gcagaatatg ctgattcaat gctgaaagcg ggtaaaccag aaacgcttga tagtcgccca    660
   gtggggacag gcccttttgt ttttgtggat tataaaacag atcaagccat acaatatgtt    720
   gcgcatgaaa attattggaa aggtagaaca cctttagatc gtttagtcat tagcattgtg    780
   cctgatgcaa caacacgtta tgcaaaatta caggcgggta cttgcgattt aattttattc    840
   ccaaatgtag cggatttagc caaaatgaaa accgatccaa aagtacaact tttggaacaa    900
   aaaggtttga acgtagcgta tatcgcattc aataccgaaa aagcaccgtt tgataatgtc    960
   aaagtgcgcc aagcgttgaa ttacgcagtg gataaaaaag cgattattga agcggtttat   1020
   caaggcgcag gaacatcagc taaaaaccca cttcctccaa caatttggag ttataatgat   1080
   gaaatccaag attatccgta cgatccagaa aaagcaaaac aacttttagc agaagcgggt   1140
   tatccaaatg gttttgaaac tgatttctgg attcaaccta ttattcgtgc ttctaatcca   1200
   aatccaaaac gtatggctga attaattatg gcggattggg cgaaaattgg tgtaaaaact   1260
   aacccagtga cttatgaatg ggctgattat agaaaacgag caaaagaggg agaattaact   1320
   gcgggtatct ttggttggtc tggcgacaat ggcgatcctg ataatttctt atccccatta   1380
   ttaggtagct caaatatagg taattctaat atggcacgtt tcaataattc agaatttgat   1440
   gctttactca atgaggctat tggattaacc aataaggaag aacgtgcgaa actttataaa   1500
   caagctcaag ttattgtcca taatcaagca ccttggattc cagttgccca ctctgttggt   1560
   tttgcgccac ttagtcctcg tgtaaaaggt tatgtacaaa gcccatttgg ctatgacgct   1620
   ttttatggtg tgagtgttga tggtaaataa                                    1650
<210> 6
   <211> 549
   <212> PRT
   <213> Haemophilus influenzae
<220>
   <221> SIGNAL
   <222> (1) .. (20)
<400> 6
   Met Leu Met Lys Leu Lys Ala Thr Leu Thr Leu Ala Ala Ala Thr Leu
   1               5                   10                  15
   Val Leu Ala Ala Cys Asp Gln Ser Ser Ser Ala Asn Lys Ser Thr Ala
               20                  25                  30
   Gln Thr Glu Ala Lys Ser Ser Ser Asn Asn Thr Phe Val Tyr Cys Thr
   35 40 45
   Ala Lys Ala Pro Leu Gly Phe Ser Pro Ala Leu Ile Ile Glu Gly Thr
   50 55 60
   Ser Tyr Asn Ala Ser Ser Gln Gln Val Tyr Asn Arg Leu Val Glu Phe
   65 70 75 80
   Lys Lys Gly Ser Thr Asp Ile Glu Pro Ala Leu Ala Glu Ser Trp Glu
   85 90 95
   Ile Ser Asp Asp Gly Leu Ser Tyr Thr Phe His Leu Arg Lys Gly Val
   100 105 110
   Lys Phe His Thr Thr Lys Glu Phe Thr Pro Thr Arg Asp Phe Asn Ala
   115 120 125
   Asp Asp Val Val Phe Ser Phe Gln Arg Gln Leu Asp Pro Asn His Pro
   130 135 140
   Tyr His Asn Val Ser Lys Gly Thr Tyr Pro Tyr Phe Lys Ala Met Lys
   145 150 155 160
   Phe Pro Glu Leu Leu Lys Ser Val Glu Lys Val Asp Asp Asn Thr Ile
   165 170 175
   Arg Ile Thr Leu Asn Lys Thr Asp Ala Thr Phe Leu Ala Ser Leu Gly
   180 185 190
   Met Asp Phe Ile Ser Ile Tyr Ser Ala Glu Tyr Ala Asp Ser Met Leu
   195 200 205
   Lys Ala Gly Lys Pro Glu Thr Leu Asp Ser Arg Pro Val Gly Thr Gly
   210 215 220
   Pro Phe Val Phe Val Asp Tyr Lys Thr Asp Gln Ala Ile Gln Tyr Val
   225 230 235 240
   Ala His Glu Asn Tyr Trp Lys Gly Arg Thr Pro Leu Asp Arg Leu Val
   245 250 255
   Ile Ser Ile Val Pro Asp Ala Thr Thr Arg Tyr Ala Lys Leu Gln Ala
   260 265 270
   Gly Thr Cys Asp Leu Ile Leu Phe Pro Asn Val Ala Asp Leu Ala Lys
   275 280 285
   Met Lys Thr Asp Pro Lys Val Gln Leu Leu Glu Gln Lys Gly Leu Asn
   290 295 300
   Val Ala Tyr Ile Ala Phe Asn Thr Glu Lys Ala Pro Phe Asp Asn Val
   305 310 315 320
   Lys Val Arg Gln Ala Leu Asn Tyr Ala Val Asp Lys Lys Ala Ile Ile
   325 330 335
   Glu Ala Val Tyr Gln Gly Ala Gly Thr Ser Ala Lys Asn Pro Leu Pro
   340 345 350
   Pro Thr Ile Trp Ser Tyr Asn Asp Glu Ile Gln Asp Tyr Pro Tyr Asp
   355 360 365
   Pro Glu Lys Ala Lys Gln Leu Leu Ala Glu Ala Gly Tyr Pro Asn Gly
   370 375 380
   Phe Glu Thr Asp Phe Trp Ile Gln Pro Ile Ile Arg Ala Ser Asn Pro
   385 390 395 400
   Asn Pro Lys Arg Met Ala Glu Leu Ile Met Ala Asp Trp Ala Lys Ile
   405 410 415
   Gly Val Lys Thr Asn Pro Val Thr Tyr Glu Trp Ala Asp Tyr Arg Lys
   420 425 430
   Arg Ala Lys Glu Gly Glu Leu Thr Ala Gly Ile Phe Gly Trp Ser Gly
   435 440 445
   Asp Asn Gly Asp Pro Asp Asn Phe Leu Ser Pro Leu Leu Gly Ser Ser
   450 455 460
   Asn Ile Gly Asn Ser Asn Met Ala Arg Phe Asn Asn Ser Glu Phe Asp
   465 470 475 480
   Ala Leu Leu Asn Glu Ala Ile Gly Leu Thr Asn Lys Glu Glu Arg Ala
   485 490 495
   Lys Leu Tyr Lys Gln Ala Gln Val Ile Val His Asn Gln Ala Pro Trp
   500 505 510
   Ile Pro Val Ala His Ser Val Gly Phe Ala Pro Leu Ser Pro Arg Val
   515 520 525
   Lys Gly Tyr Val Gln Ser Pro Phe Gly Tyr Asp Ala Phe Tyr Gly Val
   530 535 540
   Ser Val Asp Gly Lys
   545
<210> 7
   <211> 1974
   <212> DNA
   <213> Haemophilus influenzae
<400> 7
   atgatgaaca gacgtcattt tattcaaatt ggtgcgacca gtattcttgc attaagtgca     60
   aaccgttttg cgatggcaaa aggaaagagc gatgttgatt tacgcattgt ggcaacaaca    120
   gatgttcata gtttcttgac cgattttgac tattataaag atgcaccaac cgataaattc    180
   ggttttactc gtgcggcaag ccttattcgt caagcacgtg ctaaagtaaa aaatagcgta    240
   ttggtagata atggggattt aattcaaggt aatccaattg cagactatca agccgcacaa    300
   ggctataaag agggtaaatc taaccctgcg gtcgattgtt taaatgcgat gaattatgaa    360
   gtgggtacat taggtaacca cgaatttaac tatggtctca attatcttgc agatgccatc    420
   aaacaagcta aattcccaat tgtgaatgct aacgtagtaa aagcggggac agaagaacct    480
   tatttcacac cttatgtcat tcaagaaaaa tctgtggtgg ataataatgg caaaacacat    540
   aaattaaaaa ttggttacat cggttttgtg ccaccgcaaa ttatggtttg ggataaagca    600
   aaccttcaag gcaaagttga aacccgtgat attgtaaaaa cagcgcaaaa atatgtacct    660
   gaaatgaaga aaaaaggggc tgatattgtc gttgcattag ctcacactgg tccatctgat    720
   gaaccttatc aagaaggtgc tgaaaactca gcattctatc tagcggatgt tccacacatt    780
   gatgcggtta tttttggtca ctcacaccgt ttattcccaa ataaagaatt cgcaaaatca    840
   ccaaatgcag acatcgtaaa tggtaccgta aaaggcgtac cagaaagtat ggctggttac    900
   tgggcaaata atatcagcgt ggtggattta ggcttaacag aacacaaggg taaatggatc    960
   gtgacttctg gcaaagctgt gcttcgtcca atttatgatg tagaaaccaa aaaagcactt   1020
   gcaaaaaatg acccagaaat taccgcactt ttaaaaccag ttcacgaagc aacacgtaaa   1080
   tatgtttctc aacctatcgg caaagccaca gacaatatgt atagttacct tgcattaata   1140
   caagatgatc caaccattca aattgtaaac caagcacaaa aagcgtatgt agagaaagtt   1200
   gcaccaagtg ttgcagcaat ggctggctta cctattttaa gtgcaggtgc tccatttaaa   1260
   gcgggtggtc gtaaaaatga cccaacgggc tataccgaag taaataaagg tgaattaact   1320
   ttccgtaatg cagcagactt atacctttat ccaaatacgc tagtggttgt taaagcgaca   1380
   ggcgaacaat taaaagaatg gttagaatgt agtgctggta tgtttaaaca aattgaccct   1440
   acaagtgata aaccacaatc attaatcgac tgggaaggtt tccgcactta taactttgat   1500
   gtgattgatg gtgtcaatta tgaatacgat ctaaccaaac cagctcgtta cgatggcgaa   1560
   tgtaagttaa tcaacccaga atcgcatcgt gtagtgaatc tcacttatca aggtaaacca   1620
   gttgatccaa aagcagaatt tttgattgca acgaataact atcgtgctta cggcaataaa   1680
   ttcccaggta ctggcgatca acatattgtt tacgcatcac cagatgaaag ccgtcaaatt   1740
   ttggctgatt atattaaagc aaccagtgag aaagagggtt cagtcaatcc aaatgcggat   1800
   aaaaactggc gttttgtgcc tatcacaggt aacgataaat tagatgtccg ttttgaaaca   1860
   tcgccaagcg aacaagcggc gaaatttatc gcagaaaaag cacaataccc aatgaaacaa   1920
   gtgggtactg acgaaatcgg ttttgcagtg tatcaaattg atttatctaa ataa         1974
<210> 8
   <211> 657
   <212> PRT
   <213> Haemophilus influenzae
<220>
   <221> SIGNAL
   <222> (1)..(26)
<400> 8
   Met Met Asn Arg Arg His Phe Ile Gln Ile Gly Ala Thr Ser Ile Leu
   1               5                   10                  15
   Ala Leu Ser Ala Asn Arg Phe Ala Met Ala Lys Gly Lys Ser Asp Val
               20                  25                  30
   Asp Leu Arg Ile Val Ala Thr Thr Asp Val His Ser Phe Leu Thr Asp
           35                  40                  45
   Phe Asp Tyr Tyr Lys Asp Ala Pro Thr Asp Lys Phe Gly Phe Thr Arg
       50                  55                  60
   Ala Ala Ser Leu Ile Arg Gln Ala Arg Ala Lys Val Lys Asn Ser Val
   65                  70                  75                  80
   Leu Val Asp Asn Gly Asp Leu Ile Gln Gly Asn Pro Ile Ala Asp Tyr
                   85                  90                  95
   Gln Ala Ala Gln Gly Tyr Lys Glu Gly Lys Ser Asn Pro Ala Val Asp
               100                 105                 110
   Cys Leu Asn Ala Met Asn Tyr Glu Val Gly Thr Leu Gly Asn His Glu
           115                 120                 125
   Phe Asn Tyr Gly Leu Asn Tyr Leu Ala Asp Ala Ile Lys Gln Ala Lys
       130                 135                 140
   Phe Pro Ile Val Asn Ala Asn Val Val Lys Ala Gly Thr Glu Glu Pro
   145                 150                 155                 160
   Tyr Phe Thr Pro Tyr Val Ile Gln Glu Lys Ser Val Val Asp Asn Asn
                   165                 170                 175
   Gly Lys Thr His Lys Leu Lys Ile Gly Tyr Ile Gly Phe Val Pro Pro
               180                 185                 190
   Gln Ile Met Val Trp Asp Lys Ala Asn Leu Gln Gly Lys Val Glu Thr
           195                 200                 205
   Arg Asp Ile Val Lys Thr Ala Gln Lys Tyr Val Pro Glu Met Lys Lys
       210                 215                 220
   Lys Gly Ala Asp Ile Val Val Ala Leu Ala His Thr Gly Pro Ser Asp
   225                 230                 235                 240
   Glu Pro Tyr Gln Glu Gly Ala Glu Asn Ser Ala Phe Tyr Leu Ala Asp
                   245                 250                 255
   Val Pro His Ile Asp Ala Val Ile Phe Gly His Ser His Arg Leu Phe
               260                 265                 270
   Pro Asn Lys Glu Phe Ala Lys Ser Pro Asn Ala Asp Ile Val Asn Gly
           275                 280                 285
   Thr Val Lys Gly Val Pro Glu Ser Met Ala Gly Tyr Trp Ala Asn Asn
           290                 295                 300
   Ile Ser Val Val Asp Leu Gly Leu Thr Glu His Lys Gly Lys Trp Ile
   305                 310                 315                 320
   Val Thr Ser Gly Lys Ala Val Leu Arg Pro Ile Tyr Asp Val Glu Thr
                   325                 330                 335
   Lys Lys Ala Leu Ala Lys Asn Asp Pro Glu Ile Thr Ala Leu Leu Lys
               340                 345                 350
   Pro Val His Glu Ala Thr Arg Lys Tyr Val Ser Gln Pro Ile Gly Lys
           355                 360                 365
   Ala Thr Asp Asn Met Tyr Ser Tyr Leu Ala Leu Ile Gln Asp Asp Pro
       370                 375                 380
   Thr Ile Gln Ile Val Asn Gln Ala Gln Lys Ala Tyr Val Glu Lys Val
   385                 390                 395                 400
   Ala Pro Ser Val Ala Ala Met Ala Gly Leu Pro Ile Leu Ser Ala Gly
                   405                 410                 415
   Ala Pro Phe Lys Ala Gly Gly Arg Lys Asn Asp Pro Thr Gly Tyr Thr
               420                 425                 430
   Glu Val Asn Lys Gly Glu Leu Thr Phe Arg Asn Ala Ala Asp Leu Tyr
           435                 440                 445
   Leu Tyr Pro Asn Thr Leu Val Val Val Lys Ala Thr Gly Glu Gln Leu
       450                 455                 460
   Lys Glu Trp Leu Glu Cys Ser Ala Gly Met Phe Lys Gln Ile Asp Pro
   465                 470                 475                 480
   Thr Ser Asp Lys Pro Gln Ser Leu Ile Asp Trp Glu Gly Phe Arg Thr
                   485                 490                 495
   Tyr Asn Phe Asp Val Ile Asp Gly Val Asn Tyr Glu Tyr Asp Leu Thr
               500                 505                 510
   Lys Pro Ala Arg Tyr Asp Gly Glu Cys Lys Leu Ile Asn Pro Glu Ser
           515                 520                 525
   His Arg Val Val Asn Leu Thr Tyr Gln Gly Lys Pro Val Asp Pro Lys
       530                 535                 540
   Ala Glu Phe Leu Ile Ala Thr Asn Asn Tyr Arg Ala Tyr Gly Asn Lys
   545                 550                 555                 560
   Phe Pro Gly Thr Gly Asp Gln His Ile Val Tyr Ala Ser Pro Asp Glu
                   565                 570                 575
   Ser Arg Gln Ile Leu Ala Asp Tyr Ile Lys Ala Thr Ser Glu Lys Glu
               580                 585                 590
   Gly Ser Val Asn Pro Asn Ala Asp Lys Asn Trp Arg Phe Val Pro Ile
           595                 600                 605
   Thr Gly Asn Asp Lys Leu Asp Val Arg Phe Glu Thr Ser Pro Ser Glu
       610                 615                 620
   Gln Ala Ala Lys Phe Ile Ala Glu Lys Ala Gln Tyr Pro Met Lys Gln
   625                 630                 635                 640
   Val Gly Thr Asp Glu Ile Gly Phe Ala Val Tyr Gln Ile Asp Leu Ser
                   645                 650                 655
   Lys
<210> 9
   <211> 483
   <212> DNA
   <213> Haemophilus influenzae
<400> 9
   atgaaaaaaa ttattttaac attatcactt gggttactta ccgcttggtc tgctcaaatc      60
   caaaaggctg aacaaaatga tgtgaagctg gcaccgccga ctgatgtacg aagcggatat     120
   atacgtttgg taaagaatgt gaattattac atcgatagtg aatcgatctg ggtggataac     180
   caagagccac aaattgtaca ttttgataca gtggtgaatt tagataaggg attgtatgtt     240
   tatcctgagc ctaaacgtta tgcacgttct gttcgtcagt ataagatttt gaattgtgca     300
   aattatcatt taactcaagt acgaactgat ttctatgatg aattttgggg acagggtttg     360
   cgggcagcac ctaaaaagca aaagaaacat acgttaagtt taacacctga tacaacgctt     420
   tataatgctg ctcagattat ttgtgcaaat tatggtaaag cattttcagt tgataaaaaa     480
   taa                                                                   483
<210> 10
   <211> 160
   <212> PRT
   <213> Haemophilus influenzae
<220>
   <221> SIGNAL
   <222> (1)..(20)
<400> 10
   Met Lys Lys Ile Ile Leu Thr Leu Ser Leu Gly Leu Leu Thr Ala Trp
   1               5                   10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Thr Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 11
   <211> 939
   <212> DNA
   <213> Haemophilus influenzae
<400> 11
   gtgccttctg atttagtcgc acagttctct aaagaaactg gcatcgaggt aatttattct     60
   acctttgaaa gcaacgaaga aatgtatgcg aaacttaaac ttactcagaa tacgggttct    120
   ggctatgatt tggtgtttcc atcaagttac tacgtaaata aaatgattaa ggaaaagatg    180
   cttcaaccga ttgatcaaag taaattgacc aatattcatc aaatccctaa acatttatta    240
   aataaagaat tcgacccaga aaataaatat tcattacctt acgtttacgg cttaactggt    300
   attgaagtta atgcggatga gattgatcct aaaaccatta ccagctgggc agatttatgg    360
   aaacctgaat ttaaaggcaa agttctcatg accagtgatg cgcgtgaagt gttccacgta    420
   gcattattac tggatggtaa atcgccaaat accaccaatg aagaagacat caaaacagct    480
   tatgagcgtt tagaaaaact gttaccaaat gtagcgactt ttaactctga ttctcctgaa    540
   gtcccttacg tacaaggcga agttgcaatt ggtatgattt ggaatggttc agcttattta    600
   gcgcagaaag aaaatcaatc tcttcaattt gtgtatccaa aagaaggtgc aattttctgg    660
   atggataact atgcgattcc gactacagca aaaaatgttg aaggtgcaca taagttcatt    720
   gacttcttac ttcgccctga aaatgcaaaa attgttatcg aacgcatggg cttttctatg    780
   ccaaataatg gggcgaaaac attgctaagc gcggaagttg ctaatgatcc aaaattattt    840
   ccaccagcgg aagaagtgga aaaaggaatt atgcaaggcg atgtaggaga agcggtcgat    900
   atttatgaaa aatattggag taaattaaaa actaattag                           939
<210> 12
   <211> 312
   <212> PRT
   <213> Haemophilus influenzae
<400> 12
   Met Pro Ser Asp Leu Val Ala Gln Phe Ser Lys Glu Thr Gly Ile Glu
   1               5                   10                  15
   Val Ile Tyr Ser Thr Phe Glu Ser Asn Glu Glu Met Tyr Ala Lys Leu
               20                  25                  30
   Lys Leu Thr Gln Asn Thr Gly Ser Gly Tyr Asp Leu Val Phe Pro Ser
           35                  40                  45
   Ser Tyr Tyr Val Asn Lys Met Ile Lys Glu Lys Met Leu Gln Pro Ile
       50                  55                  60
   Asp Gln Ser Lys Leu Thr Asn Ile His Gln Ile Pro Lys His Leu Leu
   65                  70                  75                  80
   Asn Lys Glu Phe Asp Pro Glu Asn Lys Tyr Ser Leu Pro Tyr Val Tyr
                   85                  90                  95
   Gly Leu Thr Gly Ile Glu Val Asn Ala Asp Glu Ile Asp Pro Lys Thr
               100                 105                 110
   Ile Thr Ser Trp Ala Asp Leu Trp Lys Pro Glu Phe Lys Gly Lys Val
           115                 120                 125
   Leu Met Thr Ser Asp Ala Arg Glu Val Phe His Val Ala Leu Leu Leu
       130                 135                 140
   Asp Gly Lys Ser Pro Asn Thr Thr Asn Glu Glu Asp Ile Lys Thr Ala
   145                 150                 155                 160
   Tyr Glu Arg Leu Glu Lys Leu Leu Pro Asn Val Ala Thr Phe Asn Ser
                   165                 170                 175
   Asp Ser Pro Glu Val Pro Tyr Val Gln Gly Glu Val Ala Ile Gly Met
               180                 185                 190
   Ile Trp Asn Gly Ser Ala Tyr Leu Ala Gln Lys Glu Asn Gln Ser Leu
           195                 200                 205
   Gln Phe Val Tyr Pro Lys Glu Gly Ala Ile Phe Trp Met Asp Asn Tyr
       210                 215                 220
   Ala Ile Pro Thr Thr Ala Lys Asn Val Glu Gly Ala His Lys Phe Ile
   225                 230                 235                 240
   Asp Phe Leu Leu Arg Pro Glu Asn Ala Lys Ile Val Ile Glu Arg Met
                   245                 250                 255
   Gly Phe Ser Met Pro Asn Asn Gly Ala Lys Thr Leu Leu Ser Ala Glu
               260                 265                 270
   Val Ala Asn Asp Pro Lys Leu Phe Pro Pro Ala Glu Glu Val Glu Lys
           275                 280                 285
   Gly Ile Met Gln Gly Asp Val Gly Glu Ala Val Asp Ile Tyr Glu Lys
       290                 295                 300
   Tyr Trp Ser Lys Leu Lys Thr Asn
   305                 310
<210> 13
   <211> 1041
   <212> DNA
   <213> Haemophilus influenzae
<400> 13
   atgaagaaat ttttaattgc gattttactt ttaattttga ttttagcggg cgcggcgagt    60
   tttgcttatt acaaaatgac tgaatttgta aaaacaccag taaatgtgca ggcagatcaa   120
   cttttaacta ttgaacgtgg cacaacaggt tcaaaattag ccgcactttt tgagcaagaa   180
   aagttaattg ctgatgggaa attattacct tatttgctga agttgaaatc tgagctaaat   240
   aaaataaaag cagggactta ttctcttgag aatgttaaaa ctgtgcaaga tttactggat   300
   ttgcttaatt caggtaagga agtgcagttt aatgtaaaat ggattgaagg aaaaactttc   360
   aaggattggc gaaaagatct tgaaaatgca ccgcacttgg tgcaaacctt gaaagataag   420
   cgtaacgaag acattttcac attacttgac ttgccagatg tcggtcaaaa tcttgaacta   480
   aaaaatgtgg aaggttggct ttatcctgat acttataatt atacgcctaa atccacagac   540
   ttagagctac ttaagcgatc ggctgagcaa atgaaaaagg cgttaaataa ggcttggaac   600
   gagcgtgacg atgatttacc tttagcgaat ccttatgaaa tgttgatttt ggcttctatc   660
   gtagaaaaag aaacgggtat tgctaatgag agagcaaaag tggcctctgt atttatcaac   720
   cgtttaaaag caaaaatgaa attacagacg gatccaacgg taatttatgg tatgggcgaa   780
   aattacaatg ggaatattcg caaaaaagat ttagaaaccc taacgcctta taatacgtat   840
   gtgattgatg gattaccgcc aacacctatt gcgatgccaa gcgaaagttc gttacaagct   900
   gttgcaaaac ctgaaaaaac agatttttat tattttgtgg cagatggttc tggtgggcat   960
   aaatttaccc gaaatttgaa tgaacataat aaagcggtgc aagaatattt acgttggtat  1020
   cgcagtcaga aaaatgccaa a                                            1041
<210> 14
   <211> 347
   <212> PRT
   <213> Haemophilus influenzae
<220>
   <221> SIGNAL
   <222> (1)..(19)
<400> 14
   Met Lys Lys Phe Leu Ile Ala Ile Leu Leu Leu Ile Leu Ile Leu Ala
   1               5                   10                  15
   Gly Ala Ala Ser Phe Ala Tyr Tyr Lys Met Thr Glu Phe Val Lys Thr
               20                  25                  30
   Pro Val Asn Val Gln Ala Asp Gln Leu Leu Thr Ile Glu Arg Gly Thr
           35                  40                  45
   Thr Gly Ser Lys Leu Ala Ala Leu Phe Glu Gln Glu Lys Leu Ile Ala
       50                  55                  60
   Asp Gly Lys Leu Leu Pro Tyr Leu Leu Lys Leu Lys Ser Glu Leu Asn
   65                  70                  75                  80
   Lys Ile Lys Ala Gly Thr Tyr Ser Leu Glu Asn Val Lys Thr Val Gln
                   85                  90                  95
   Asp Leu Leu Asp Leu Leu Asn Ser Gly Lys Glu Val Gln Phe Asn Val
               100                 105                 110
   Lys Trp Ile Glu Gly Lys Thr Phe Lys Asp Trp Arg Lys Asp Leu Glu
           115                 120                 125
   Asn Ala Pro His Leu Val Gln Thr Leu Lys Asp Lys Arg Asn Glu Asp
       130                 135                 140
   Ile Phe Thr Leu Leu Asp Leu Pro Asp Val Gly Gln Asn Leu Glu Leu
   145                 150                 155                 160
   Lys Asn Val Glu Gly Trp Leu Tyr Pro Asp Thr Tyr Asn Tyr Thr Pro
                   165                 170                 175
   Lys Ser Thr Asp Leu Glu Leu Leu Lys Arg Ser Ala Glu Gln Met Lys
               180                 185                 190
   Lys Ala Leu Asn Lys Ala Trp Asn Glu Arg Asp Asp Asp Leu Pro Leu
           195                 200                 205
   Ala Asn Pro Tyr Glu Met Leu Ile Leu Ala Ser Ile Val Glu Lys Glu
       210                 215                 220
   Thr Gly Ile Ala Asn Glu Arg Ala Lys Val Ala Ser Val Phe Ile Asn
   225                 230                 235                 240
   Arg Leu Lys Ala Lys Met Lys Leu Gln Thr Asp Pro Thr Val Ile Tyr
                   245                 250                 255
   Gly Met Gly Glu Asn Tyr Asn Gly Asn Ile Arg Lys Lys Asp Leu Glu
               260                 265                 270
   Thr Leu Thr Pro Tyr Asn Thr Tyr Val Ile Asp Gly Leu Pro Pro Thr
           275                 280                 285
   Pro Ile Ala Met Pro Ser Glu Ser Ser Leu Gln Ala Val Ala Lys Pro
       290                 295                 300
   Glu Lys Thr Asp Phe Tyr Tyr Phe Val Ala Asp Gly Ser Gly Gly His
   305                 310                 315                     320
   Lys Phe Thr Arg Asn Leu Asn Glu His Asn Lys Ala Val Gln Glu Tyr
                   325                 330                 335
   Leu Arg Trp Tyr Arg Ser Gln Lys Asn Ala Lys
               340                 345
<210> 15
   <211> 942
   <212> DNA
   <213> Haemophilus influenzae
<400> 15
   atgaaaatga aaaaatttat tctaaaatct tttttattgg ctactttggg atgtgtcgct     60
   ttcgcttcta tggcacaagc ggaggaacgt gtcgtagcga cagtggatgg tattcctgtt    120
   ttagaaagcc aagtgcgtgc caatatgggt aaaaaaggtg atcgccaaag tgcgattgat    180
   aaaattattg atgatatttt ggtgcaaaaa gcagttcaag aatcgggagt caaaattgat    240
   cctcgtgaaa ttgatcatat tgtggaagac actgcggcaa gaaatggctt aacttatggt    300
   caatttttgg atgcgttaga ttaccaaggc atttcattaa atgcatttcg tcagcaaatt    360
   gccaatcaaa tggtgatggg ggctgtacgc aacaaagcta ttcaagaaag cattgatgta    420
   acgcgtgaag aagttgtcgc actcggtcaa aaaatgttag aagaagcgaa agaaaagggt    480
   acggcgcaga aagttatggg gaaagagtat gaagttcgcc atattttgtt aaaacttaat    540
   ccattgttaa atgatgctca agcaaaaaaa caattagcta aaattcgttc tgatattatt    600
   gcaggtaaaa caacttttgc tgatgccgca ttaaaatatt ctaaagatta tttatcgggt    660
   gcgaatggcg gtagtttagg ttatgcgttc ccagaaactt atgcaccaca gtttgcgcaa    720
   accgtcgtga aaagtaaaca aggtgtgatt tctgcaccat ttaaaactga gtttggttgg    780
   catattttgg aagtaactgg cgtacgtgat ggcgatctta cagcagaagc ctacacacaa    840
   aaagcatatg aacgtttagt aaatactcaa ttacaagatg cgacgaacga ttgggttaaa    900
   gcattgcgta aaagagcgaa tattcagtat tttaataaat aa                      942
<210> 16
   <211> 313
   <212> PRT
   <213> Haemophilus influenzae
<220>
   <221> SIGNAL
   <222> (1)..(27)
<400> 16
   Met Lys Met Lys Lys Phe Ile Leu Lys Ser Phe Leu Leu Ala Thr Leu
   1               5                   10                  15
   Gly Cys Val Ala Phe Ala Ser Met Ala Gln Ala Glu Glu Arg Val Val
               20                  25                  30
   Ala Thr Val Asp Gly Ile Pro Val Leu Glu Ser Gln Val Arg Ala Asn
           35                  40                  45
   Met Gly Lys Lys Gly Asp Arg Gln Ser Ala Ile Asp Lys Ile Ile Asp
       50                  55                  60
   Asp Ile Leu Val Gln Lys Ala Val Gln Glu Ser Gly Val Lys Ile Asp
   65                  70                  75                  80
   Pro Arg Glu Ile Asp His Ile Val Glu Asp Thr Ala Ala Arg Asn Gly
                   85                  90                  95
   Leu Thr Tyr Gly Gln Phe Leu Asp Ala Leu Asp Tyr Gln Gly Ile Ser
               100                 105                 110
   Leu Asn Ala Phe Arg Gln Gln Ile Ala Asn Gln Met Val Met Gly Ala
           115                 120                 125
   Val Arg Asn Lys Ala Ile Gln Glu Ser Ile Asp Val Thr Arg Glu Glu
       130                 135                 140
   Val Val Ala Leu Gly Gln Lys Met Leu Glu Glu Ala Lys Glu Lys Gly
   145                 150                 155                 160
   Thr Ala Gln Lys Val Met Gly Lys Glu Tyr Glu Val Arg His Ile Leu
                   165                 170                 175
   Leu Lys Leu Asn Pro Leu Leu Asn Asp Ala Gln Ala Lys Lys Gln Leu
               180                 185                 190
   Ala Lys Ile Arg Ser Asp Ile Ile Ala Gly Lys Thr Thr Phe Ala Asp
           195                 200                 205
   Ala Ala Leu Lys Tyr Ser Lys Asp Tyr Leu Ser Gly Ala Asn Gly Gly
       210                 215                 220
   Ser Leu Gly Tyr Ala Phe Pro Glu Thr Tyr Ala Pro Gln Phe Ala Gln
   225                 230                 235                 240
   Thr Val Val Lys Ser Lys Gln Gly Val Ile Ser Ala Pro Phe Lys Thr
                   245                 250                 255
   Glu Phe Gly Trp His Ile Leu Glu Val Thr Gly Val Arg Asp Gly Asp
               260                 265                 270
   Leu Thr Ala Glu Ala Tyr Thr Gln Lys Ala Tyr Glu Arg Leu Val Asn
           275                 280                 285
   Thr Gln Leu Gln Asp Ala Thr Asn Asp Trp Val Lys Ala Leu Arg Lys
       290                 295                 300
   Arg Ala Asn Ile Gln Tyr Phe Asn Lys
   305                 310
<210> 17
   <211> 487
   <212> DNA
   <213> Haemophilus influenzae
<400> 17
   atgactatgt ttaaaaaaat ctctgtttta ttttttacgt taatattggc aggttgttct     60
   tcttggtcat cggttactaa ctatattcca tttatgggga acgataaaaa agtgattgat    120
   ttagataaag ataaaatcga tcaaaaatcc tatgcagcgg cttatgaagc gactgtggcg    180
   acatataaag gtcgcgtgaa tgaaaatttc tttgtagata attttgcaag tggtgcgaat    240
   gactggtatt taggtcgtat tttagttcct gtgaaacaaa ttcaggataa actttataca    300
   ggcggtcatg attctgatat atatgcttat tatagcggcg tgttacatgc ggaagcattg    360
   caagctaacc ttaaacgttt aagtgtaagc tgttgggaaa aagtagattc gcaaagtatg    420
   gctcaaggta tttatgatgc gatgcgtgat tagtgaagcg ctgctgaaag cctgtacatc    480
   taaataa                                                              487
<210> 18
   <211> 178
   <212> PRT
   <213> Haemophilus influenzae
<220>
   <221> SIGNAL
   <222> (1)..(23)
<400> 18
   Met Thr Met Phe Lys Lys Ile Ser Val Leu Phe Phe Thr Leu Ile Leu
   1               5                   10                  15
   Ala Gly Cys Ser Ser Trp Ser Ser Val Thr Asn Tyr Ile Pro Phe Met
               20                  25                  30
   Gly Asn Asp Lys Lys Val Ile Asp Leu Asp Lys Asp Lys Ile Asp Gln
           35                  40                  45
   Lys Ser Tyr Ala Ala Ala Tyr Glu Ala Thr Val Ala Thr Tyr Lys Gly
       50                  55                  60
   Arg Val Asn Glu Asn Phe Phe Val Asp Asn Phe Ala Ser Gly Ala Asn
   65                  70                  75                  80
   Asp Trp Tyr Leu Gly Arg Ile Leu Val Pro Val Lys Gln Ile Gln Asp
                   85                  90                  95
   Lys Leu Tyr Thr Gly Gly His Asp Ser Asp Ile Tyr Ala Tyr Tyr Ser
               100                 105                 110
   Gly Val Leu His Ala Glu Ala Leu Gln Ala Asn Leu Lys Arg Leu Ser
           115                 120                 125
   Val Ser Cys Trp Glu Lys Val Asp Ser Gln Ser Met Ala Gln Gly Ile
       130                 135                 140
   Tyr Asp Ala Met Arg Asp Leu Gln Lys Gly Glu Ala Arg Gly Glu Asn
   145                 150                 155                 160
   Asp Glu Tyr Ile Val Gln Gly Ser Glu Ala Leu Leu Lys Ala Cys Thr
                   165                 170                 175
   Ser Lys
<210> 19
   <211> 1728
   <212> DNA
   <213> Haemophilus influenzae
<400> 19
   atgtctattc tattacaagg cgaacgtttt aaaaaacgtt taatgccaat tttattgtca 60
   atggctttag ctggctgttc aaatctactt ggtagcaatt tcacgcaaac cttacaaaaa 120
   gatgcaaatg caagttctga attttatata aacaaattag ggcaaacgca agaacttgaa 180
   gatcaacaaa cctataaatt gctcgcggct cgagtgttaa tccgtgaaaa taaggttgaa 240
   caatcggcag cgttattgag ggaattaggc gaattaaatg atgcgcaaaa attagatcgt 300
   gcattaattg aagcgagaat ttctgccgca aaaaatgcca atgaagtcgc acaaaatcaa 360
   ttacgtgcat tggatttaaa taaactaagc ccgtcacaaa aatctcgtta ttacgaaacc 420
   ttagctattg ttgccgaaaa ccgtaaagac atgattgaag cggtaaaagc gcggatagaa 480
   atggataaga atttaacaga tgtacaacgt cgtcaagata atattgataa aacttgggct 540
   ttattgcgtt cagcgaatac tggcgttatt aataatgcct ctgatgaagg taatgcagct 600
   ttaggcggtt ggctaacatt aatcaaagcc tacaacgatt atattcgtca gcctgtacaa 660
   ttaagccaag ccttacaaag ttggaaaaat gcttatccaa atcatgcagc cgcaacgttg     720
   ttcccaaaag aattgcttac attgcttaat ttccaacaaa cgaatgtgtc acaaattggt     780
   ttactcttgc cattaagtgg agatggacaa attcttggta caaccattca atcgggtttt     840
   aacgacgcga aaggtaactc aaccattcca gtgcaagtgt ttgatacctc aatgaattct     900
   gtccaagata tcattgcgca agcaaaacaa gcggggatta aaaccttagt cggcccatta     960
   ctaaaacaaa atcttgatgt gattttagcg gatcctgctc aaattcaagg tatggatgtg    1020
   cttgcattaa atgccacacc acattctcgt gcgattcctc aactttgtta ttacggactt    1080
   tctccagaag atgaagctga atctgccgcc aataaaatgt ggaacgatgg cgtgcgtaat    1140
   ccacttgtcg caatgccgca aaatgattta ggacaacgcg taggcaatgc ctttaatgta    1200
   cgttggcagc aattagcagg tactgatgcg aatatccgtt actacaattt gcctgcggat    1260
   gtgacctatt tcgttcaaga aaataactca aatacaaccg cactttatgc cgtagcaagt    1320
   ccaactgaac tggcagaaat gaaaggttat ttaacaaata tcgtacctaa tttagcgatt    1380
   tatgccagtt ctcgagcaag cgcaagtgcg acaaacacta ataccgactt catcgcacag    1440
   atgaacggtg tacagtttag tgatattcca ttttttaaag ataccaattc tccacaatat     1500
   cagaagttag caaaatccac ggggggtgaa tatcaattga tgcgtttata tgcaatgggt     1560
   gcagatgcgt ggttgctcat taatcaattt aatgaattac gccaagtgcc aggctatcgc    1620
   ttgagtggct taacagggat tttaagtgct gataccaact gtaatgttga acgcgatatg    1680
   acttggtatc aatatcaaga tggtgcaatt gtaccagttg caaactaa                 1728
<210> 20
   <211> 575
   <212> PRT
   <213> Haemophilus influenzae
<220>
   <221> SIGNAL
   <222> (1)..(31)
<400> 20
   Met Ser Ile Leu Leu Gln Gly Glu Arg Phe Lys Lys Arg Leu Met Pro
   1               5                   10                  15
   Ile Leu Leu Ser Met Ala Leu Ala Gly Cys Ser Asn Leu Leu Gly Ser
               20                  25                  30
   Asn Phe Thr Gln Thr Leu Gln Lys Asp Ala Asn Ala Ser Ser Glu Phe
           35                  40                  45
   Tyr Ile Asn Lys Leu Gly Gln Thr Gln Glu Leu Glu Asp Gln Gln Thr
       50                  55                  60
   Tyr Lys Leu Leu Ala Ala Arg Val Leu Ile Arg Glu Asn Lys Val Glu
   65                  70                  75                  80
   Gln Ser Ala Ala Leu Leu Arg Glu Leu Gly Glu Leu Asn Asp Ala Gln
                   85                  90                  95
   Lys Leu Asp Arg Ala Leu Ile Glu Ala Arg Ile Ser Ala Ala Lys Asn
               100                 105                 110
   Ala Asn Glu Val Ala Gln Asn Gln Leu Arg Ala Leu Asp Leu Asn Lys
           115                 120                 125
   Leu Ser Pro Ser Gln Lys Ser Arg Tyr Tyr Glu Thr Leu Ala Ile Val
       130                 135                 140
   Ala Glu Asn Arg Lys Asp Met Ile Glu Ala Val Lys Ala Arg Ile Glu
   145                 150                 155                 160
   Met Asp Lys Asn Leu Thr Asp Val Gln Arg Arg Gln Asp Asn Ile Asp
                   165                 170                 175
   Lys Thr Trp Ala Leu Leu Arg Ser Ala Asn Thr Gly Val Ile Asn Asn
               180                 185                 190
   Ala Ser Asp Glu Gly Asn Ala Ala Leu Gly Gly Trp Leu Thr Leu Ile
           195                 200                 205
   Lys Ala Tyr Asn Asp Tyr Ile Arg Gln Pro Val Gln Leu Ser Gln Ala
       210                 215                 220
   Leu Gln Ser Trp Lys Asn Ala Tyr Pro Asn His Ala Ala Ala Thr Leu
   225                 230                 235                 240
   Phe Pro Lys Glu Leu Leu Thr Leu Leu Asn Phe Gln Gln Thr Asn Val
                   245                 250                 255
   Ser Gln Ile Gly Leu Leu Leu Pro Leu Ser Gly Asp Gly Gln Ile Leu
               260                 265                 270
   Gly Thr Thr Ile Gln Ser Gly Phe Asn Asp Ala Lys Gly Asn Ser Thr
           275                 280                 285
   Ile Pro Val Gln Val Phe Asp Thr Ser Met Asn Ser Val Gln Asp Ile
       290                 295                 300
   Ile Ala Gln Ala Lys Gln Ala Gly Ile Lys Thr Leu Val Gly Pro Leu
   305                 310                 315                 320
   Leu Lys Gln Asn Leu Asp Val Ile Leu Ala Asp Pro Ala Gln Ile Gln
                   325                 330                 335
   Gly Met Asp Val Leu Ala Leu Asn Ala Thr Pro His Ser Arg Ala Ile
               340                 345                 350
   Pro Gln Leu Cys Tyr Tyr Gly Leu Ser Pro Glu Asp Glu Ala Glu Ser
           355                 360                 365
   Ala Ala Asn Lys Met Trp Asn Asp Gly Val Arg Asn Pro Leu Val Ala
       370                 375                 380
   Met Pro Gln Asn Asp Leu Gly Gln Arg Val Gly Asn Ala Phe Asn Val
   385                 390                 395                 400
   Arg Trp Gln Gln Leu Ala Gly Thr Asp Ala Asn Ile Arg Tyr Tyr Asn
                   405                 410                 415
   Leu Pro Ala Asp Val Thr Tyr Phe Val Gln Glu Asn Asn Ser Asn Thr
               420                 425                 430
   Thr Ala Leu Tyr Ala Val Ala Ser Pro Thr Glu Leu Ala Glu Met Lys
           435                 440                 445
   Gly Tyr Leu Thr Asn Ile Val Pro Asn Leu Ala Ile Tyr Ala Ser Ser
       450                 455                 460
   Arg Ala Ser Ala Ser Ala Thr Asn Thr Asn Thr Asp Phe Ile Ala Gln
   465                 470                 475                 480
   Met Asn Gly Val Gln Phe Ser Asp Ile Pro Phe Phe Lys Asp Thr Asn
                   485                 490                 495
   Ser Pro Gln Tyr Gln Lys Leu Ala Lys Ser Thr Gly Gly Glu Tyr Gln
               500                 505                 510
   Leu Met Arg Leu Tyr Ala Met Gly Ala Asp Ala Trp Leu Leu Ile Asn
           515                 520                 525
   Gln Phe Asn Glu Leu Arg Gln Val Pro Gly Tyr Arg Leu Ser Gly Leu
       530                 535                 540
   Thr Gly Ile Leu Ser Ala Asp Thr Asn Cys Asn Val Glu Arg Asp Met
   545                 550                 555                 560
   Thr Trp Tyr Gln Tyr Gln Asp Gly Ala Ile Val Pro Val Ala Asn
                   565                 570                 575
<210> 21
   <211> 3336
   <212> DNA
   <213> Haemophilus influenzae
<400> 21
   atgattagga aacttatgaa aacacctcca ttttttaccg cactttttgc ctcggcaata     60
   ttcaccctta gtgtttcaca aggggtatta ggggcaaatt caacaaatgt attaccaacg    120
   gagcaatcgc ttaaagccga tttggctaac gctcaaaaaa tgagtgaggg ggaagcaaaa    180
   aagaggttgt tagcagaatt acaaacatca attgatttat tgcagcaaat tcaagctcaa    240
   caaaaaatca atgatgcatt gcaaacaaca ttaagccatt cagagagtga aattagaaag    300
   aataatgcag aaattcaagc attaaaaaaa caacaagaaa cagcaacaag tactgattat    360
   aatgcacagt cacaggacga tttacaaaat agtctcgcta agttaaatga tcaattacaa    420
   gatacgcaaa acgcattagg cgcggcaaat gcacaattgg cagggcaaaa ttctatttct    480
   gaacgagctc aagcggcatt aacggaaaat gttgtacgca ctcagcaaat taatcaacaa    540
   ttggccaata atgatattgg tagcgcatta cgaaaacaat atcagattga attacaactg    600
   attgatttaa agaatagtta taatcaaaat ttattaaaaa ataatgatca gctttcttta    660
   ctttatcaaa gtcgttacga cctattgaat ttacgtttgc aggtgcaaca acaaaatatt    720
   attgcgattc aagaagtcat taatcaaaaa agtcttcagc aatcacaaaa tcaactagaa    780
   caagctcagc agcaacagca aaaaactgtg caaaatgatt acattcaaaa agaacttgat    840
   cgcaatgcac agcttggtca gtatctatta caacaaacag aaaaagcgaa ttcattaact    900
   caagatgagt taagaatgcg gaatatttta gatagcctca ctcaaactca acatactatt    960
   gatgaacaaa ttagtgcatt acaaggcacg ttagttcttt cacgtattat ccagcaacaa   1020
   aaacaaaaat taccgactaa cttaaatatt caaggtttat caaaacaaat tgccgatttg   1080
   cgtgtgcata tttttgacat tactcaaaaa cgcaatgaac tttatgatct agataactat   1140
   attaataaag ttgaaagtga agatggaaaa caatttactg aggcagaaag aacgcaagtt   1200
   aaaacgctat taactgagcg tcggaaaatg acatctgatc tgattaaatc cttaaataat   1260
   caattaaatc tcgcgatttc tttggaatta acacagcagc aaattacaca aatcagtgat   1320
   caaattcaat ctaaattaga gcagcaaagt ttttgggtga aaagtaataa tcccattaat   1380
   ttagattgga taaaaatgct tccaagagct ttaattgaac agtttaatgg tatgttgaaa   1440
   aaattaggtt ttccaactaa ttatgacaat ttaccttatt tacttatgta tttcttaggg   1500
   ttatttattg taggcggtgc aatttttaaa tttaaaaatc gtattaaaca acaattaaac   1560
   aaaattaatc gtgaaataca tcgcttagat acagatagtc agtggagtac gccacttgcc   1620
   ctgttattaa ctgcgttttt aacgctttct agtacacttt ggtttttagc agtttgccaa   1680
   atgatcggct tctttttctt caaaaatcca gaagaatttt ggcattggtc atttagtatg   1740
   gcgagttatt ggtggttctt tacattttgg atttcattgt tccgtccaaa tggtattttt   1800
   gttaatcatt ttgaatcttc aaaagagaat gcacaacgtt ttcgtggtgt tatccagcgt   1860
   attattattg cgatagtatt actcttgaat acatctgttt ttagtaatgt aacagacagt   1920
   ggcttagcca atgatgtcct aggggaaatt aatactatta cggcgttaat tttctgcacg   1980
   gcgattattg ctcctcgttt taatcgagta cttcgctctt atgaacctga aacaaataaa   2040
   catcattggt taataaaaat tgtacaaatt ggtttgagat taattcctgt gggattaatc   2100
   gtacttattg ttttaggcta ttactacacc gctttaaatt taattgagca ttttattcat   2160
   tcttatattg cttggtgtgt atggtggtta gtacgtaata cgatttaccg tggtattacg   2220
   gtttcttctc gtcgtttagc acatcgccgt ttagcagaaa aacgtcgtca aaaagcactt   2280
   gaaaataatt atgaaaatat ttcctctgat gatgtggttg cagtgggaga gccagaggaa   2340
   ggtttagcgt taaatgatgt gcgtagccaa ttattacgtt ttgtagatct ctttatttgg   2400
   acagcattat tggggatttt ctactatgta tggtcagatt tagtcacagt agtgagctat   2460
   ttacgtgaaa ttacactttg gcaacaaacc acgacaaccg atgctggcac tgtaatggaa   2520
   agcattactt tatttaatct tcttgttgct ttggttatcc taggaattac ttatgtgttg   2580
   gttcgtaata tttcaggtat tttggaagta ttaattttct ctcgcgtgaa tctttcacaa   2640
   ggtacgcctt atacgattac gacattgctc acttatattt ttatcgccat tggtggtgcg   2700
   tgggcatttg caaccttagg aatgtcttgg tcaaaattgc aatggttatt tgccgcactt   2760
   tccgttggtc ttggttttgg tatgcaagaa atttttgcaa actttgtgtc gggcattatt   2820
   ttgctatttg aacgcccaat ccgagttggc gatgtcgtaa ccattaatgg agtgagcggt   2880
   actgtagcga aaattcgtat tcgtgcaatt acattaattg attttgatcg caaagaaatt   2940
   attgtgccaa ataaatcttt tgtgacaggt caagtaacca attgggcatt atctagcacg   3000
   atgacacgtt tagtgattag cgttggtgtt gcttatggtt ctgatttaac actcgttcgt   3060
   caattattac ttcaagcagc tgatgaacag ccgactgttt tacgcgatcc taaaccatct   3120
   gcttattttt taacttttgg tgcaagtact ttggatcacg aattacgtgt ttatgtagaa   3180
   caagttggag atcgtaccag tactacagat gctattaatc gccgtattaa tgaattattt   3240
   gcagaacata atattgatat tgcctttaat caattagatg tatttatcaa aaataatgac   3300
   actggcgaag aaattccttt tgttgatgta aaaaaa                             3336
<210> 22
   <211> 1112
   <212> PRT
   <213> Haemophilus influenzae
<220>
   <221> SIGNAL
   <222> (1)..(31)
<400> 22
   Met Ile Arg Lys Leu Met Lys Thr Pro Pro Phe Phe Thr Ala Leu Phe
   1               5                   10                  15
   Ala Ser Ala Ile Phe Thr Leu Ser Val Ser Gln Gly Val Leu Gly Ala
               20                  25                  30
   Asn Ser Thr Asn Val Leu Pro Thr Glu Gln Ser Leu Lys Ala Asp Leu
           35                  40                  45
   Ala Asn Ala Gln Lys Met Ser Glu Gly Glu Ala Lys Lys Arg Leu Leu
       50                  55                  60
   Ala Glu Leu Gln Thr Ser Ile Asp Leu Leu Gln Gln Ile Gln Ala Gln
   65                  70                  75                  80
   Gln Lys Ile Asn Asp Ala Leu Gln Thr Thr Leu Ser His Ser Glu Ser
                   85                  90                  95
   Glu Ile Arg Lys Asn Asn Ala Glu Ile Gln Ala Leu Lys Lys Gln Gln
               100                 105                 110
   Glu Thr Ala Thr Ser Thr Asp Tyr Asn Ala Gln Ser Gln Asp Asp Leu
           115                 120                 125
   Gln Asn Ser Leu Ala Lys Leu Asn Asp Gln Leu Gln Asp Thr Gln Asn
       130                 135                 140
   Ala Leu Gly Ala Ala Asn Ala Gln Leu Ala Gly Gln Asn Ser Ile Ser
   145                 150                 155                 160
   Glu Arg Ala Gln Ala Ala Leu Thr Glu Asn Val Val Arg Thr Gln Gln
                   165                 170                 175
   Ile Asn Gln Gln Leu Ala Asn Asn Asp Ile Gly Ser Ala Leu Arg Lys
               180                 185                 190
   Gln Tyr Gln Ile Glu Leu Gln Leu Ile Asp Leu Lys Asn Ser Tyr Asn
           195                 200                 205
   Gln Asn Leu Leu Lys Asn Asn Asp Gln Leu Ser Leu Leu Tyr Gln Ser
       210                 215                 220
   Arg Tyr Asp Leu Leu Asn Leu Arg Leu Gln Val Gln Gln Gln Asn Ile
   225                 230                 235                 240
   Ile Ala Ile Gln Glu Val Ile Asn Gln Lys Ser Leu Gln Gln Ser Gln
                   245                 250                 255
   Asn Gln Leu Glu Gln Ala Gln Gln Gln Gln Gln Lys Thr Val Gln Asn
               260                 265                 270
   Asp Tyr Ile Gln Lys Glu Leu Asp Arg Asn Ala Gln Leu Gly Gln Tyr
           275                 280                 285
   Leu Leu Gln Gln Thr Glu Lys Ala Asn Ser Leu Thr Gln Asp Glu Leu
       290                 295                 300
   Arg Met Arg Asn Ile Leu Asp Ser Leu Thr Gln Thr Gln His Thr Ile
   305                 310                 315                 320
   Asp Glu Gln Ile Ser Ala Leu Gln Gly Thr Leu Val Leu Ser Arg Ile
                   325                 330                 335
   Ile Gln Gln Gln Lys Gln Lys Leu Pro Thr Asn Leu Asn Ile Gln Gly
               340                 345                 350
   Leu Ser Lys Gln Ile Ala Asp Leu Arg Val His Ile Phe Asp Ile Thr
           355                 360                 365
   Gln Lys Arg Asn Glu Leu Tyr Asp Leu Asp Asn Tyr Ile Asn Lys Val
       370                 375                 380
   Glu Ser Glu Asp Gly Lys Gln Phe Thr Glu Ala Glu Arg Thr Gln Val
   385                 390                 395                 400
   Lys Thr Leu Leu Thr Glu Arg Arg Lys Met Thr Ser Asp Leu Ile Lys
                   405                 410                 415
   Ser Leu Asn Asn Gln Leu Asn Leu Ala Ile Ser Leu Glu Leu Thr Gln
               420                 425                 430
   Gln Gln Ile Thr Gln Ile Ser Asp Gln Ile Gln Ser Lys Leu Glu Gln
           435                 440                 445
   Gln Ser Phe Trp Val Lys Ser Asn Asn Pro Ile Asn Leu Asp Trp Ile
       450                 455                 460
   Lys Met Leu Pro Arg Ala Leu Ile Glu Gln Phe Asn Gly Met Leu Lys
   465                 470                 475                 480
   Lys Leu Gly Phe Pro Thr Asn Tyr Asp Asn Leu Pro Tyr Leu Leu Met
                   485                 490                 495
   Tyr Phe Leu Gly Leu Phe Ile Val Gly Gly Ala Ile Phe Lys Phe Lys
               500                 505                 510
   Asn Arg Ile Lys Gln Gln Leu Asn Lys Ile Asn Arg Glu Ile His Arg
           515                 520                 525
   Leu Asp Thr Asp Ser Gln Trp Ser Thr Pro Leu Ala Leu Leu Leu Thr
       530                 535                 540
   Ala Phe Leu Thr Leu Ser Ser Thr Leu Trp Phe Leu Ala Val Cys Gln
   545                 550                 555                 560
   Met Ile Gly Phe Phe Phe Phe Lys Asn Pro Glu Glu Phe Trp His Trp
                   565                 570                 575
   Ser Phe Ser Met Ala Ser Tyr Trp Trp Phe Phe Thr Phe Trp Ile Ser
               580                 585                 590
   Leu Phe Arg Pro Asn Gly Ile Phe Val Asn His Phe Glu Ser Ser Lys
           595                 600                 605
   Glu Asn Ala Gln Arg Phe Arg Gly Val Ile Gln Arg Ile Ile Ile Ala
       610                 615                 620
   Ile Val Leu Leu Leu Asn Thr Ser Val Phe Ser Asn Val Thr Asp Ser
   625                 630                 635                 640
   Gly Leu Ala Asn Asp Val Leu Gly Glu Ile Asn Thr Ile Thr Ala Leu
                   645                 650                 655
   Ile Phe Cys Thr Ala Ile Ile Ala Pro Arg Phe Asn Arg Val Leu Arg
               660                 665                 670
   Ser Tyr Glu Pro Glu Thr Asn Lys His His Trp Leu Ile Lys Ile Val
           675                 680                 685
   Gln Ile Gly Leu Arg Leu Ile Pro Val Gly Leu Ile Val Leu Ile Val
       690                 695                 700
   Leu Gly Tyr Tyr Tyr Thr Ala Leu Asn Leu Ile Glu His Phe Ile His
   705                 710                 715                 720
   Ser Tyr Ile Ala Trp Cys Val Trp Trp Leu Val Arg Asn Thr Ile Tyr
                   725                 730                 735
   Arg Gly Ile Thr Val Ser Ser Arg Arg Leu Ala His Arg Arg Leu Ala
               740                 745                 750
   Glu Lys Arg Arg Gln Lys Ala Leu Glu Asn Asn Tyr Glu Asn Ile Ser
           755                 760                 765
   Ser Asp Asp Val Val Ala Val Gly Glu Pro Glu Glu Gly Leu Ala Leu
       770                 775                 780
   Asn Asp Val Arg Ser Gln Leu Leu Arg Phe Val Asp Leu Phe Ile Trp
   785                 790                 795                 800
   Thr Ala Leu Leu Gly Ile Phe Tyr Tyr Val Trp Ser Asp Leu Val Thr
                   805                 810                 815
   Val Val Ser Tyr Leu Arg Glu Ile Thr Leu Trp Gln Gln Thr Thr Thr
               820                 825                 830
   Thr Asp Ala Gly Thr Val Met Glu Ser Ile Thr Leu Phe Asn Leu Leu
           835                 840                 845
   Val Ala Leu Val Ile Leu Gly Ile Thr Tyr Val Leu Val Arg Asn Ile
       850                 855                 860
   Ser Gly Ile Leu Glu Val Leu Ile Phe Ser Arg Val Asn Leu Ser Gln
   865                 870                 875                 880
   Gly Thr Pro Tyr Thr Ile Thr Thr Leu Leu Thr Tyr Ile Phe Ile Ala
                   885                 890                 895
   Ile Gly Gly Ala Trp Ala Phe Ala Thr Leu Gly Met Ser Trp Ser Lys
               900                 905                 910
   Leu Gln Trp Leu Phe Ala Ala Leu Ser Val Gly Leu Gly Phe Gly Met
           915                 920                 925
   Gln Glu Ile Phe Ala Asn Phe Val Ser Gly Ile Ile Leu Leu Phe Glu
       930                 935                 940
   Arg Pro Ile Arg Val Gly Asp Val Val Thr Ile Asn Gly Val Ser Gly
   945                 950                 955                 960
   Thr Val Ala Lys Ile Arg Ile Arg Ala Ile Thr Leu Ile Asp Phe Asp
                   965                 970                 975
   Arg Lys Glu Ile Ile Val Pro Asn Lys Ser Phe Val Thr Gly Gln Val
               980                 985                 990
   Thr Asn Trp Ala Leu Ser Ser Thr  Met Thr Arg Leu Val  Ile Ser Val
           995                 1000                1005
   Gly Val  Ala Tyr Gly Ser Asp  Leu Thr Leu Val Arg  Gln Leu Leu
       1010                1015                1020
   Leu Gln  Ala Ala Asp Glu Gln  Pro Thr Val Leu Arg  Asp Pro Lys
       1025                1030                1035
   Pro Ser  Ala Tyr Phe Leu Thr  Phe Gly Ala Ser Thr  Leu Asp His
       1040                1045                1050
   Glu Leu  Arg Val Tyr Val Glu  Gln Val Gly Asp Arg  Thr Ser Thr
       1055                1060                1065
   Thr Asp  Ala Ile Asn Arg Arg  Ile Asn Glu Leu Phe  Ala Glu His
       1070                1075                1080
   Asn Ile  Asp Ile Ala Phe Asn  Gln Leu Asp Val Phe  Ile Lys Asn
       1085                1090                1095
   Asn Asp  Thr Gly Glu Glu Ile  Pro Phe Val Asp Val  Lys Lys
       1100                1105                1110
<210> 23
   <211> 816
   <212> DNA
   <213> Haemophilus influenzae
<400> 23
   atgaaattaa aacaactttt tgcaatcact gcaatcgcat cagctctcgt tttaacaggc    60
   tgtaaagaag acaaaaaacc tgaagcagca gcaccgctta aaatcaaagt aggcgtgatg    120
   tctggccctg agcatcaagt tgcagaaatt gcagcaaaag tcgctaaaga aaaatatggt    180
   ttagacgttc aattcgttga attcaatgac tacgcattac caaatgaagc tgtagctaaa    240
   ggtgatttag atgcaaacgc aatgcaacat aaaccttatt tagatgaaga tgcaaaagcg    300
   aaaaatttaa ataacttagt tatcgtgggt aatactttcg tctatccatt agcgggttat    360
   tctaaaaaaa tcaaaaatgt gaatgaatta caagacggtg ctaaagttgt tgttcctaac    420
   gatccaacaa accgtggtcg tgcattaatt cttcttgaaa aacaaggttt aatcaaatta    480
   aaagatgcaa ataaccttct ttcaactgta ttagatattg ttgaaaatcc gaaaaaatta    540
   aacatcactg aagtagatac ttctgttgcg gcacgcgcat tagacgacgt tgatttagct    600
   gtagtaaaca atacttatgc gggtcaagta ggcttaaatg ctcaagatga cggtgtattt    660
   gtagaagata aagattctcc atatgtgaac attatcgttt ctcgtaccga taacaaagac    720
   agcaaagctg ttcaagattt cgtaaaatct taccaaacag aagaagttta ccaagaagct    780
   caaaaacact ttaaagatgg cgttgtaaaa ggttgg                              816
<210> 24
   <211> 272
   <212> PRT
   <213> Haemophilus influenzae
<220>
   <221> SIGNAL
   <222> (1)..(13)
<400> 24
   Met Lys Leu Lys Gln Leu Phe Ala Ile Thr Ala Ile Ala Ser Ala Leu
   1               5                   10                  15
   Val Leu Thr Gly Cys Lys Glu Asp Lys Lys Pro Glu Ala Ala Ala Pro
               20                  25                  30
   Leu Lys Ile Lys Val Gly Val Met Ser Gly Pro Glu His Gln Val Ala
           35                  40                  45
   Glu Ile Ala Ala Lys Val Ala Lys Glu Lys Tyr Gly Leu Asp Val Gln
       50                  55                  60
   Phe Val Glu Phe Asn Asp Tyr Ala Leu Pro Asn Glu Ala Val Ala Lys
   65                  70                  75                  80
   Gly Asp Leu Asp Ala Asn Ala Met Gln His Lys Pro Tyr Leu Asp Glu
                   85                  90                  95
   Asp Ala Lys Ala Lys Asn Leu Asn Asn Leu Val Ile Val Gly Asn Thr
               100                 105                 110
   Phe Val Tyr Pro Leu Ala Gly Tyr Ser Lys Lys Ile Lys Asn Val Asn
           115                 120                 125
   Glu Leu Gln Asp Gly Ala Lys Val Val Val Pro Asn Asp Pro Thr Asn
       130                 135                 140
   Arg Gly Arg Ala Leu Ile Leu Leu Glu Lys Gln Gly Leu Ile Lys Leu
   145                 150                 155                 160
   Lys Asp Ala Asn Asn Leu Leu Ser Thr Val Leu Asp Ile Val Glu Asn
                   165                 170                 175
   Pro Lys Lys Leu Asn Ile Thr Glu Val Asp Thr Ser Val Ala Ala Arg
               180                 185                 190
   Ala Leu Asp Asp Val Asp Leu Ala Val Val Asn Asn Thr Tyr Ala Gly
           195                 200                 205
   Gln Val Gly Leu Asn Ala Gln Asp Asp Gly Val Phe Val Glu Asp Lys
       210                 215                 220
   Asp Ser Pro Tyr Val Asn Ile Ile Val Ser Arg Thr Asp Asn Lys Asp
   225                 230                 235                 240
   Ser Lys Ala Val Gln Asp Phe Val Lys Ser Tyr Gln Thr Glu Glu Val
                   245                 250                 255
   Tyr Gln Glu Ala Gln Lys His Phe Lys Asp Gly Val Val Lys Gly Trp
               260                 265                 270
<210> 25
   <211> 33
   <212> DNA
   <213> Artificial Sequence
<220>
   <223> Primer HAMJ342
<400> 25 caaggcgttt tcatatgcct gtcattcggc agg 33
<210> 26
   <211> 45
   <212> DNA
   <213> Artificial sequence
<220>
   <223> Primer HAMJ343
<400> 26 ctaattgacc tcgagttttg ctgcttttaa ttcttgataa tattg 45
<210> 27
   <211> 41
   <212> DNA
   <213> Artificial Sequence
<220>
   <223> Primer HAMJ345
<400> 27 gtaaggaaac atacatatga aaaaactttt aaaaattagt g 41
<210> 28
   <211> 40
   <212> DNA
   <213> Artificial Sequence
<220>
   <223> Primer HAMJ344
<400> 28 ttagagactc gagtttagct aaacattcta tgtagctatc 40
<210> 29
   <211> 38
   <212> DNA
   <213> Artificial Sequence
<220>
   <223> Primer HAM3348
<400> 29 cctaacattg acatatgctt atgaaactaa aagcaaca 38
<210> 30
   <211> 34
   <212> DNA
   <213> Artificial Sequence
<220>
   <223> Primer HAMJ349
<400> 30 tcaatatctc gagtttacca tcaacactca cacc 34
<210> 31
   <211> 42
   <212> DNA
   <213> Artificial Sequence
<220>
   <223> Primer HAMJ346
<400> 31 ctaataagga aaaccatatg atgaacagac gtcattttat tc 42
<210> 32
   <211> 39
   <212> DNA
   <213> Artificial sequence
<220>
   <223> Primer HAMJ399
<400> 32 gacctagctc gagtttagat aaatcaattt gatacactg 39
<210> 33
   <211> 41
   <212> DNA
   <213> Artificial Sequence
<220>
   <223> Primer HAMJ376
<400> 33 cataaggagt aacatatgaa aaaaattatt ttaacattat c 41
<210> 34
   <211> 31
   <212> DNA
   <213> Artificial sequence
<220>
   <223> Primer HAMJ377
<400> 34 gtgcagattc tcgagttttt tatcaactga a 31
<210> 35
   <211> 33
   <212> DNA
   <213> Artificial Sequence
<220>
   <223> Primer HAMJ460
<400> 35 ctggacagac catatgcctt ctgatttagt cgc 33
<210> 36
   <211> 36
   <212> DNA
   <213> Artificial Sequence
<220>
   <223> Primer HAMJ461
<400> 36 ctaaattgag ctcgagatta gtttttaatt tactcc 36
<210> 37
   <211> 38
   <212> DNA
   <213> Artificial sequence
<220>
   <223> Primer HAMJ458
<400> 37 ctttcttata gcatatgaag aaatttttaa ttgcgatt 38
<210> 38
   <211> 27
   <212> DNA
   <213> Artificial Sequence
<220>
   <223> Primer HAMJ459
<400> 38 cttccgcact cgagtttggc atttttc 27
<210> 39
   <211> 35
   <212> DNA
   <213> Artificial sequence
<220>
   <223> Primer HAMJ477
<400> 39 ggaaggagct agcatgaaaa tgaaaaaatt tattc 35
<210> 40
   <211> 29
   <212> DNA
   <213> Artificial Sequence
<220>
   <223> Primer HAMJ478
<400> 40 ttgatactct cgagtttatt aaaatactg 29
<210> 41
   <211> 37
   <212> DNA
   <213> Artificial Sequence
<220>
   <223> Primer HAMJ481
<400> 41 ctttaatcac atatgactat gtttaaaaaa atctctg 37
<210> 42
   <211> 30
   <212> DNA
   <213> Artificial Sequence
<220>
   <223> Primer HAMJ482
<400> 42 caccaatctc gagtttagat gtacaggctt 30
<210> 43
   <211> 31
   <212> DNA
   <213> Artificial Sequence
<220>
   <223> Primer HAMJ78
<400> 43 acccgcatgg ctgttcaaat ctacttggta g 31
<210> 44
   <211> 31
   <212> DNA
   <213> Artificial Sequence
<220>
   <223> Primer HAMJ79
<400> 44 actcgtcgac ttagtttgca actggtacaa t 31
<210> 45
   <211> 35
   <212> DNA
   <213> Artificial Sequence
<220>
   <223> Primer HAMJ414
<400> 45 gattagggaa aacatatgat taggaaactt atgaa 35
<210> 46
   <211> 31
   <212> DNA
   <213> Artificial Sequence
<220>
   <223> Primer HAMJ415
<400> 46 ccataatttg ctcgagtttt tttacatcaa c 31
<210> 47
   <211> 35
   <212> DNA
   <213> Artificial sequence
<220>
   <223> Primer HAMJ450
<400> 47 ggaaggagct agcatgaaat taaaacaact ttttg 35
<210> 48
   <211> 27
   <212> DNA
   <213> Artificial sequence
<220>
   <223> Primer HAMJ411
<400> 48 gtgcggtagc tcgagccaac cttttac 27
<210> 49
   <211> 379
   <212> PRT
   <213> _{H}aemophilus influenzae
<400> 49
   Met Pro Val Ile Arg Gln Val Val Phe Tyr Asp Ser Leu Thr Gly Glu
   1               5                   10                  15
   Gln Thr Lys Met Lys Lys Phe Ala Gly Leu Ile Thr Ala Ser Phe Val
               20                  25                  30
   Ala Ala Thr Leu Thr Ala Cys Asn Asp Lys Asp Ala Lys Gln Glu Thr
           35                  40                  45
   Ala Lys Ala Thr Ala Ala Ala Asn Asp Thr Val Tyr Leu Tyr Thr Trp
       50                  55                  60
   Thr Glu Tyr Val Pro Asp Gly Leu Leu Asp Glu Phe Thr Lys Glu Thr
   65                  70                  75                  80
   Gly Ile Lys Val Ile Val Ser Ser Leu Glu Ser Asn Glu Thr Met Tyr
                   85                  90                  95
   Ala Lys Leu Lys Thr Gln Gly Glu Ser Gly Gly Tyr Asp Val Ile Ala
               100                 105                 110
   Pro Ser Asn Tyr Phe Val Ser Lys Met Ala Arg Glu Gly Met Leu Lys
           115                 120                 125
   Glu Leu Asp His Ser Lys Leu Pro Val Leu Lys Glu Leu Asp Pro Asp
       130                 135                 140
   Trp Leu Asn Lys Pro Tyr Asp Lys Gly Asn Lys Tyr Ser Leu Pro Gln
   145                 150                 155                 160
   Leu Leu Gly Ala Pro Gly Ile Ala Phe Asn Thr Asn Thr Tyr Lys Gly
                   165                 170                 175
   Glu Gln Phe Thr Ser Trp Ala Asp Leu Trp Lys Pro Glu Phe Ala Asn
               180                 185                 190
   Lys Val Gln Leu Leu Asp Asp Ala Arg Glu Val Phe Asn Ile Ala Leu
           195                 200                 205
   Leu Lys Ile Gly Gln Asp Pro Asn Thr Gln Asp Pro Ala Ile Ile Lys
       210                 215                 220
   Gln Ala Tyr Glu Glu Leu Leu Lys Leu Arg Pro Asn Val Leu Ser Phe
   225                 230                 235                 240
   Asn Ser Asp Asn Pro Ala Asn Ser Phe Ile Ser Gly Glu Val Glu Val
                   245                 250                 255
   Gly Gln Leu Trp Asn Gly Ser Val Arg Ile Ala Lys Lys Glu Lys Ala
               260                 265                 270
   Pro Leu Asn Met Val Phe Pro Lys Glu Gly Pro Val Leu Trp Val Asp
           275                 280                 285
   Thr Leu Ala Ile Pro Val Thr Ala Lys Asn Pro Glu Gly Ala His Lys
       290                 295                 300
   Leu Ile Asn Tyr Met Leu Gly Lys Lys Thr Ala Glu Lys Leu Thr Leu
   305                 310                 315                 320
   Ala Ile Gly Tyr Pro Thr Ser Asn Ile Glu Ala Lys Lys Ala Leu Pro
                   325                 330                 335
   Lys Glu Ile Thr Glu Asp Pro Ala Ile Tyr Pro Ser Ala Asp Ile Leu
               340                 345                 350
   Lys Asn Ser His Trp Gln Asp Asp Val Gly Asp Ala Ile Gln Phe Tyr
           355                 360                 365
   Glu Gln Tyr Tyr Gln Glu Leu Lys Ala Ala Lys
       370                 375
<210> 50
   <211> 379
   <212> PRT
   <213> Haemophilus influenzae
<400> 50
   Met Pro Val Ile Arg Gln Val Val Phe Tyr Asp Ser Leu Thr Gly Glu
   1               5                   10                  15
   Gln Thr Lys Met Lys Lys Phe Ala Gly Leu Ile Thr Ala Ser Phe Val
               20                  25                  30
   Ala Ala Thr Leu Thr Ala Cys Asn Asp Lys Asp Ala Lys Gln Glu Thr
           35                  40                  45
   Ala Lys Ala Thr Ala Ala Ala Asn Asp Thr Val Tyr Leu Tyr Thr Trp
       50                  55                  60
   Thr Glu Tyr Val Pro Asp Gly Leu Leu Asp Glu Phe Thr Lys Glu Thr
   65                  70                  75                  80
   Gly Ile Lys Val Ile Val Ser Ser Leu Glu Ser Asn Glu Thr Met Tyr
                   85                  90                  95
   Ala Lys Leu Lys Thr Gln Gly Glu Ser Gly Gly Tyr Asp Val Ile Ala
               100                 105                 110
   Pro Ser Asn Tyr Phe Val Ser Lys Met Ala Arg Glu Gly Met Leu Lys
           115                 120                 125
   Glu Leu Asp His Ser Lys Leu Pro Val Leu Lys Glu Leu Asp Pro Asp
       130                 135                 140
   Trp Leu Asn Lys Pro Tyr Asp Lys Gly Asn Lys Tyr Ser Leu Pro Gln
   145                 150                 155                 160
   Leu Leu Gly Ala Pro Gly Ile Ala Phe Asn Thr Asn Thr Tyr Lys Gly
                   165                 170                 175
   Glu Glu Phe Thr Ser Trp Ala Asp Leu Trp Lys Pro Glu Phe Ala Asn
               180                 185                 190
   Lys Val Gln Leu Leu Asp Asp Ala Arg Glu Val Phe Asn Ile Ala Leu
           195                 200                 205
   Leu Lys Ile Gly Gln Asp Pro Asn Thr Gln Asp Pro Ala Ile Ile Lys
       210                 215                 220
   Gln Ala Tyr Glu Glu Leu Leu Lys Leu Arg Pro Asn Val Leu Ser Phe
   225                 230                 235                 240
   Asn Ser Asp Asn Pro Ala Asn Ser Phe Ile Ser Gly Glu Val Glu Val
                   245                 250                 255
   Gly Gln Leu Trp Asn Gly Ser Val Arg Ile Ala Lys Lys Glu Lys Ala
               260                 265                 270
   Pro Leu Asn Met Val Phe Pro Lys Glu Gly Pro Val Leu Trp Val Asp
           275                 280                 285
   Thr Leu Ala Ile Pro Ala Thr Ala Lys Asn Ser Glu Gly Ala His Lys
       290                 295                 300
   Leu Ile Asn Tyr Met Leu Gly Lys Lys Thr Ala Glu Lys Leu Thr Leu
   305                 310                 315                 320
   Ala Ile Gly Tyr Pro Thr Ser Asn Ile Glu Ala Lys Lys Ala Leu Pro
                   325                 330                 335
   Lys Glu Ile Thr Glu Asp Pro Ala Ile Tyr Pro Ser Ala Asp Ile Leu
               340                 345                 350
   Lys Asn Ser His Trp Gln Asp Asp Val Gly Asp Ala Ile Gln Phe Tyr
           355                 360                 365
   Glu Gln Tyr Tyr Gln Glu Leu Lys Ala Ala Lys
       370                 375
<210> 51
   <211> 379
   <212> PRT
   <213> Haemophilus influenzae
<400> 51
   Met Pro Val Ile Arg Gln Val Val Phe Tyr Asp Ser Leu Thr Gly Glu
   1               5                   10                  15
   Gln Thr Lys Met Lys Lys Phe Ala Gly Leu Ile Thr Ala Ser Phe Val
               20                  25                  30
   Ala Ala Thr Leu Thr Ala Cys Asn Asp Lys Asp Ala Lys Gln Glu Thr
           35                  40                  45
   Ala Lys Ala Thr Ala Ala Ala Asn Asp Thr Val Tyr Leu Tyr Thr Trp
       50                  55                  60
   Thr Glu Tyr Val Pro Asp Gly Leu Leu Asp Glu Phe Thr Lys Glu Thr
   65                  70                  75                  80
   Gly Ile Lys Val Ile Val Ser Ser Leu Glu Ser Asn Glu Thr Met Tyr
                   85                  90                  95
   Ala Lys Leu Lys Thr Gln Gly Glu Ser Gly Gly Tyr Asp Val Ile Ala
               100                 105                 110
   Pro Ser Asn Tyr Phe Val Ser Lys Met Ala Arg Glu Gly Met Leu Lys
           115                 120                 125
   Glu Leu Asp His Ser Lys Leu Pro Val Leu Lys Glu Leu Asp Pro Asp
       130                 135                 140
   Trp Leu Asn Lys Pro Tyr Asp Lys Gly Asn Lys Tyr Ser Leu Pro Gln
   145                 150                 155                 160
   Leu Leu Gly Ala Pro Gly Ile Ala Phe Asn Thr Asn Thr Tyr Lys Gly
                   165                 170                 175
   Glu Gln Phe Thr Ser Trp Ala Asp Leu Trp Lys Pro Glu Phe Ala Asn
               180                 185                 190
   Lys Val Gln Leu Leu Asp Asp Ala Arg Glu Val Phe Asn Ile Ala Leu
           195                 200                 205
   Leu Lys Ile Gly Gln Asp Pro Asn Thr Gln Asp Pro Ala Ile Ile Lys
       210                 215                 220
   Gln Ala Tyr Glu Glu Leu Leu Lys Leu Arg Pro Asn Val Leu Ser Phe
   225                 230                 235                 240
   Asn Ser Asp Asn Pro Ala Asn Ser Phe Ile Ser Gly Glu Val Glu Val
                   245                 250                 255
   Gly Gln Leu Trp Asn Gly Ser Val Arg Ile Ala Lys Lys Glu Lys Ala
               260                 265                 270
   Pro Leu Asn Met Val Phe Pro Lys Glu Gly Pro Val Leu Trp Val Asp
           275                 280                 285
   Thr Leu Ala Ile Pro Val Thr Ala Lys Asn Pro Glu Gly Ala His Lys
       290                 295                 300
   Leu Ile Asn Tyr Met Leu Gly Lys Lys Thr Ala Glu Lys Leu Thr Leu
   305                 310                 315                 320
   Ala Ile Gly Tyr Pro Thr Ser Asn Ile Glu Ala Lys Lys Ala Leu Pro
                   325                 330                 335
   Lys Glu Ile Thr Glu Asp Pro Ala Ile Tyr Pro Ser Ala Asp Ile Leu
               340                 345                 350
   Lys Asn Ser His Trp Gln Asp Asp Val Gly Asp Ala Ile Gln Phe Tyr
           355                 360                 365
   Glu Gln Tyr Tyr Gln Glu Leu Lys Ala Ala Lys
       370                 375

## Description

### FIELD OF THE INTENTION

The present invention is related to polypeptides of Haemophilus influenzae and corresponding DNA fragments, which may be useful to prevent, diagnose and/or treat Haemophilus influenzae infections in individuals such as humans.

### BACKGROUND OF THE INVENTION

Haemophilus influenzae is a Gram-negative rod that is found in nature only as a human pathogen. Isolates of H. influenzae can be subdivided into encapsulated and non-encapsulated forms. Encapsulated strains express one of six structurally and antigenically distinct capsular polysaccharides that are designated, types "a" to "f". Non-encapsulated strains are defined by their failure to agglutinate with antisera against the recognised H. influenzae capsular polysaccharides and are referred to as nontypeable.

Nontypeable H. influenzae strains commonly colonise the upper respiratory tract, including the nasopharynx and the posterior oropharynx. A number of surveys of healthy individuals indicate colonisation rates between 40% to 80% among both children and adults (Spinola S.M., Peacock J., Denny F.W., Smith D.L. and Cannon J.G. (1986) Epidemiology of colonisation by nontypeable Haemophilus influenzae in children : a longitudinal study. J. Infect. Dis. 154 :100-109; Trottier S., Stenberg K. and Svanborg-Eden C. (1989) Turn over of nontypeable Haemophilus influenzae in the nasopharynges of healthy children. J. Clin. Microbiol. 27 :2175-2179). Colonization with a particular strain may persist for weeks or months with most individuals by remaining asymptomatic throughout this period. The pathogenesis of disease due to nontypeable H. influenzae involves contiguous spread within the respiratory tract. Spread to adjacent areas is usually a consequence of abnormalities in either non-specific or specific host defences. So, nontypeable H. influenzae causes a variety of respiratory tract infections in children and adults including otitis, sinusitis, bronchitis and pneumonia. These infections may become chronic or recurrent in patients with bronchitis or otitis. In fact, in infants and children, nontypeable H. influenzae is a frequent cause of acute otitis media and is commonly implicated in recurrent otitis media (Harabuchi Y., Fadden H., Yamanaka N., Duffy L., Wolf J., Krystofik D. and Tonawanda/Williamsville Pediatrics. (1994) Nasopharyngeal colonisation with nontypeable Haemophilus influenzae and recurrent otitis media. J. Infect. Dis. 170 :862-866). In infants, nontypeable H. influenzae is responsible for between 27% and 37% of the first episode of otitis media by the age of 1 year (Smith-Vaughan H.C., Sriprakash K.S., Mathews J.D. and Kemp D.J. (1997) Nonencapsulated Haemophilus influenzae in aboriginal infants with otitis media : prolonged carriage of P2 porin variants and evidence for horizontal P2 gene transfer. Infect. Immun. 65 :1468-1474; Teele D.W., Klein J.O., Rosner B. and Greater Boston Otitis Media study Group. (1989) Epidemiology of otitis media during the first seven years of life in children in Greater Boston : a prospective, cohort study. J. Infect. Dis. 160 :83-94). Meningitis is sometimes caused by nontypeable H. influenzae and accounts for 1-3% of all cases. But, nontypeable H. influenzae is particularly prevalent in hosts with an underlying disease which affects the innate mucosal immune system, such as chronic obstructive pulmonary disease (COPD) and cystic fibrosis (Murphy T.F. and Sethi S. (1992) Bacterial infection in chronic obstructive pulmonary disease. Am. Rev. Respir. Dis. 146 :1067-1083; St. Geme J.W. III. (1993) Nontypeable Haemophilus influenzae disease : epidemiology, pathogenesis and prospects for prevention. Inflect. Agents Dis . 2 :1-16). Nontypeable H. influenzae strains are found predominantly during exacerbations when the sputum becomes mucopurulent. Acute infective exacerbations of chronic bronchitis play an important role in the morbidity and mortality of patients with chronic pulmonary diseases.

The etiologies of community-acquired pneumonia appear to have changed in the last decade. While Streptococcus pneumoniae remains the predominant pathogen, the proportion of cases involving other organisms has increased. H. influenzae is now often reported as being the second most common cause of pneumonia. Ten percent of H. influenzae pneumoniae cases develop into bacteremia. In developing countries pneumonia caused by nontypeable H. influenzae is apparently an important cause of morbidity and mortality in children.

WO 96/33276 discloses the entire genome of Haemophilus influenzae Rd and possible clinical uses thereof and of the polypeptides encoded thereby.

Although several H. influenzae type polysaccharide conjugated vaccines have been developed, these vaccines are ineffective against disease caused by other H. influenzae strains (Scheifele D.W., Jadavji T.P., Law B.J., Gold R., Macdonald N.E., Lebel M.H., Mills E.L., Dery P., Halperin S.A., Morris R.F., Marchessault V. and Duclos P.J. (1996) Recent trends in pediatric Haemophilus influenzae type b infections in Canada. Can. Med. Assoc. J. 154 :1041-1047; Schulte E.E., Birkhead G.S., Kondracki S.F. and Morse D.L. (1994) Patterns of Haemophilus influenzae type b invasive disease in New York State, 1987-991 : the role of vaccination requirements for day-care attendance. Pediatrics 94 :1014-1016). The identification of conserved cross-protective antigens is critical for the development of a universal vaccine against H. influenzae infection and disease. Outer membrane proteins such as P1, P2, P4, P6, PCP, OMP26 and D-15 have been identified or are currently explored as potential vaccine antigens (Foxwell A.R., Kyd J.M. and Cripps A.W. (1998) Nontypeable Haemophilus influenzae : Pathogenesis and Prevention. Microbiol. Mol. Biol. Rev. 62 :294-308). Protein D-15 is the only conserved immunogen that has been described, in the scientific literature, as being capable of conferring protection against multiple serotypes and nontypeable strains.

Therefore there remains an unmet need for H. influenzae polypeptides that may be useful to prevent, diagnose and/or treat Haemophilus influenzae infections in individuals such as humans.

### SUMMARY OF THE INVENTION

According to one aspect, the present invention provides an isolated polynucleotide encoding a polypeptide having at least 70% identity to a second polypeptide comprising a sequence chosen from SEQ ID Nos : 2, 4, 6, 8, 10, 12, 14, 16, 18, 20, 22 and 24 or fragments or analogs thereof.

According to one aspect, the present invention relates to polypeptides which comprise an amino acid sequence selected from SEQ ID Nos : 2, 4, 6, 8, 10, 12, 14, 16, 18, 20, 22 and 24 or fragments or analogs thereof.

In other aspects, there are provided polypeptides encoded by polynucleotides of the invention, pharmaceutical compositions, vectors comprising polynucleotides of the invention operably linked to an expression control region, as well as host cells transfected with said vectors and processes of producing polypeptides comprising culturing said host cells under conditions suitable for expression.

### BRIEF DESCRIPTION OF THE DRAWINGS

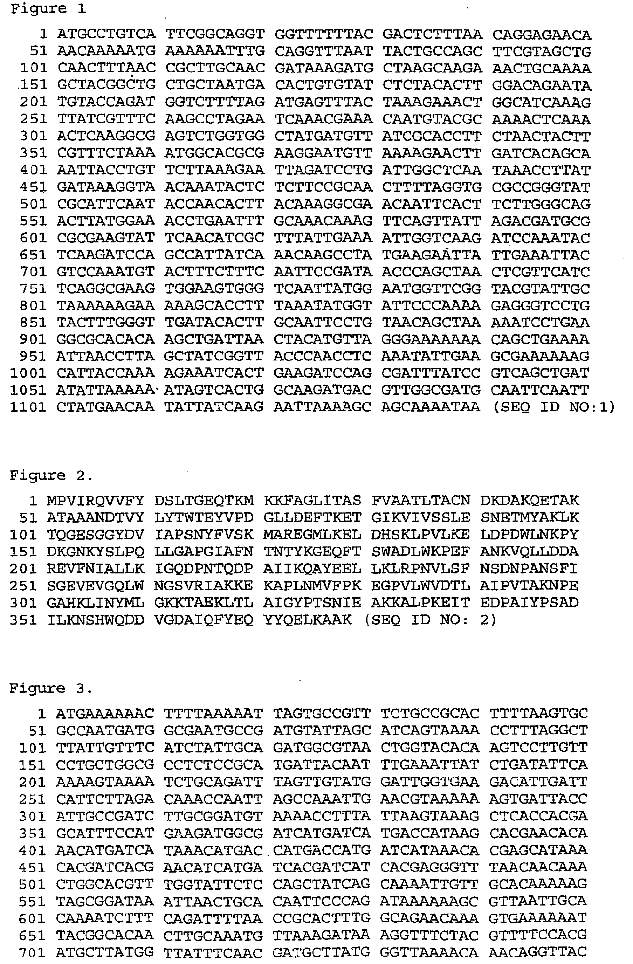

Figure 1 represents the DNA sequence of BVH-NTHI1 gene from nontypeable H. influenzae strain 12085; SEQ ID NO: 1.
Figure 2 represents the deduced amino acid sequence of the full-length BVH-NTHI1 from nontypeable H. influenzae strain 12085; SEQ ID NO: 2.
Figure 3 represents the DNA sequence of BVH-NTHI2 gene from nontypeable H. influenzae strain 12085; SEQ ID NO: 3.

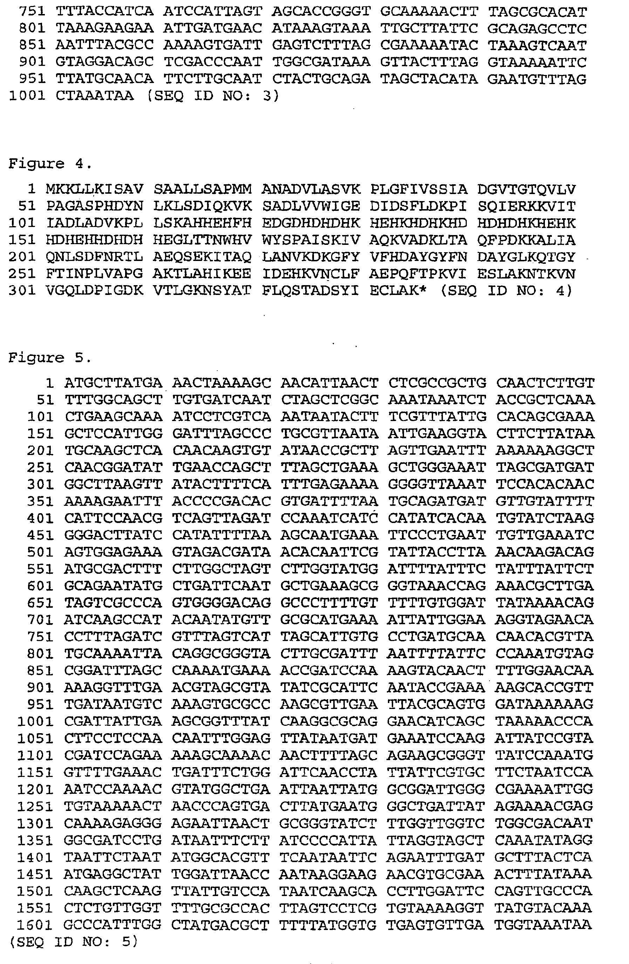

Figure 4 represents the deduced amino acid sequence of the full-length BVH-NTHI2 from nontypeable H. influenzae strain 12085; SEQ ID NO: 4.
Figure 5 represents the DNA sequence of BVH-NTHI3 gene from nontypeable H. influenzae strain 12085; SEQ ID NO: 5.

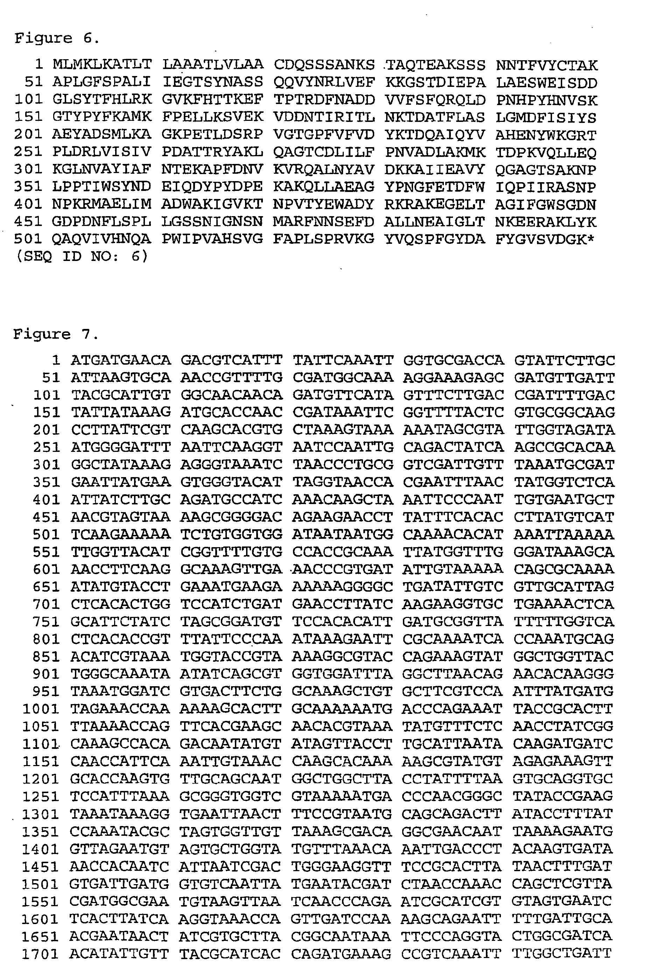

Figure 6 represents the deduced amino acid sequence of the full-length BVH-NTHI3 from nontypeable H. influenzae strain 12085; SEQ ID NO: 6.
Figure 7 represents the DNA sequence of BVH-NTHI4 gene from nontypeable H. influenzae strain 12085; SEQ ID NO: 7.

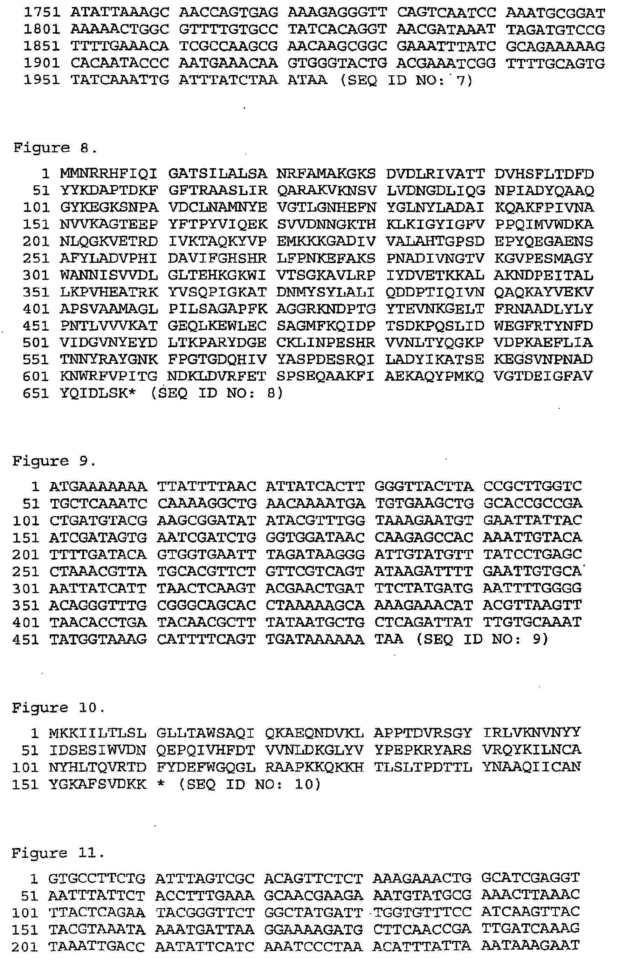

Figure 8 represents the deduced amino acid sequence of the full-length BVH-NTHI4 from nontypeable H. influenzae strain 12085; SEQ ID NO: 8.
Figure 9 represents the DNA sequence of BVH-NTHI5 gene from nontypeable H. influenzae strain 12085; SEQ ID NO: 9.
Figure 10 represents the deduced amino acid sequence of the full-length BVH-NTHI5 from nontypeable H. influenzae strain 12085; SEQ ID NO: 10.
Figure 11 represents the DNA sequence of BVH-NTHI6 gene from nontypeable H. influenzae strain 12085; SEQ ID NO: 11.

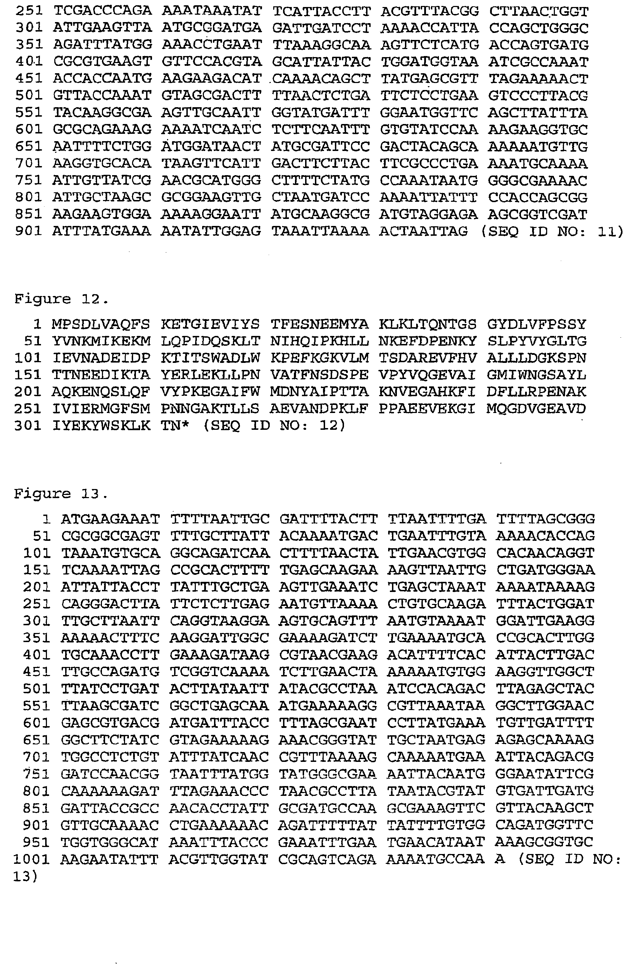

Figure 12 represents the deduced amino acid sequence of the full-length BVH-NTHI6 from nontypeable H. influenzae strain 12085; SEQ ID NO: 12.
Figure 13 represents the DNA sequence of BVH-NTHI7 gene from nontypeable H. influenzae strain 12085; SEQ ID NO: 13.

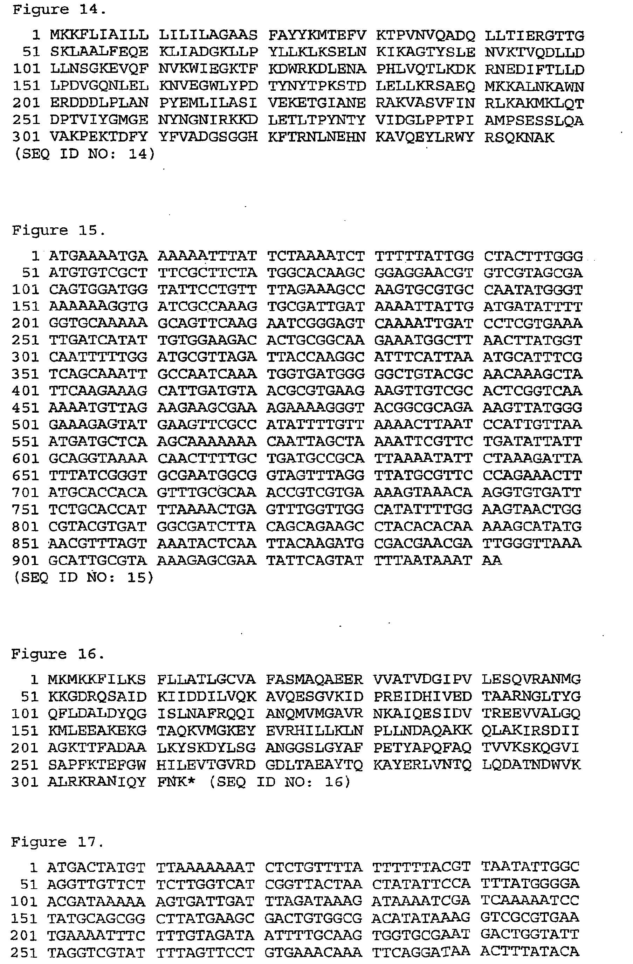

Figure 14 represents the deduced amino acid sequence of the full-length BVH-NTHI7 from nontypeable H. influenzae strain 12085; SEQ ID NO: 14.
Figure 15 represents the DNA sequence of BVH-NTHI8 gene from nontypeable H. influenzae strain 12085; SEQ ID NO: 15.
Figure 16 represents the deduced amino acid sequence of the full-length BVH-NTHI8 from nontypeable H. influenzae strain 12085; SEQ ID NO: 16.
Figure 17 represents the DNA sequence of BVH-NTHI9 gene from nontypeable H. influenzae strain 12085; SEQ ID NO: 17.

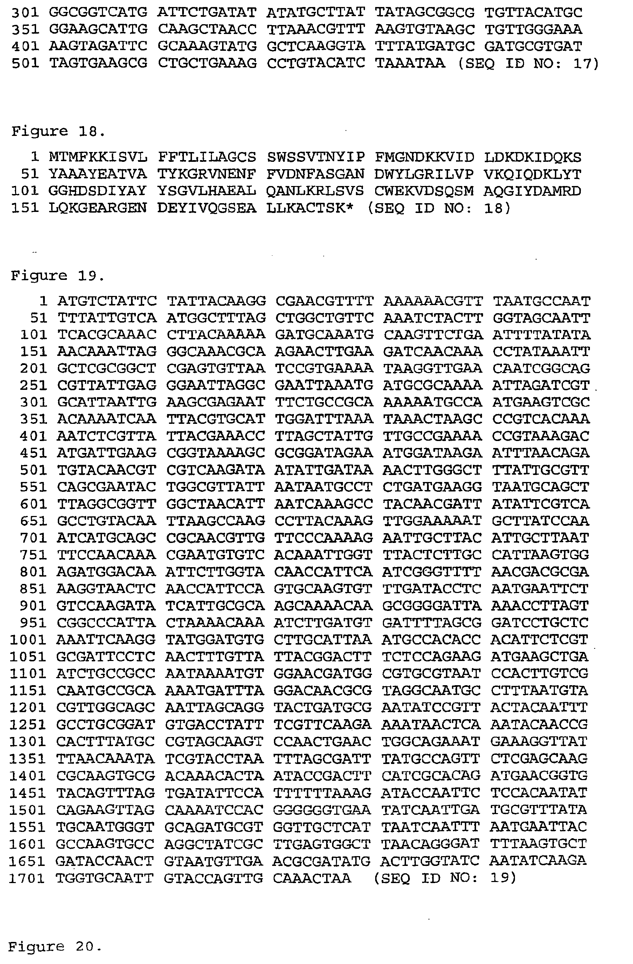

Figure 18 represents the deduced amino acid sequence of the full-length BVH-NTHI9 from nontypeable H. influenzae strain 12085; SEQ ID NO: 18.
Figure 19 represents the DNA sequence of BVH-NTHI10 gene from nontypeable H. influenzae strain 12085; SEQ ID NO: 19.
Figure 20 represents the deduced amino acid sequence of the full-length BVH-NTHI10 from nontypeable H. influenzae strain 12085; SEQ ID NO: 20.

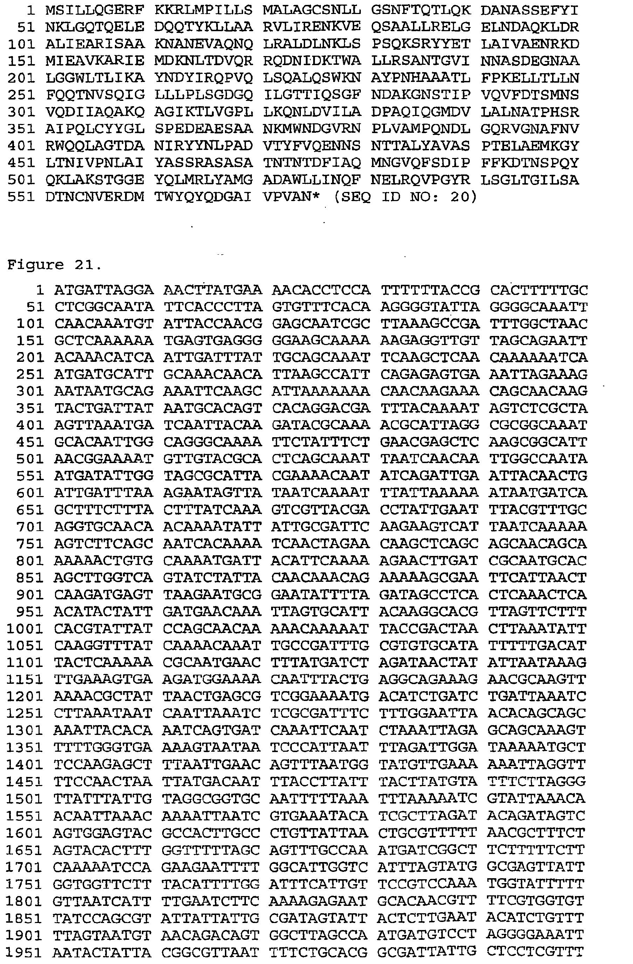

Figure 21 represents the DNA sequence of BVH-NTHI11 gene from nontypeable H. influenzae strain 12085; SEQ ID NO: 21

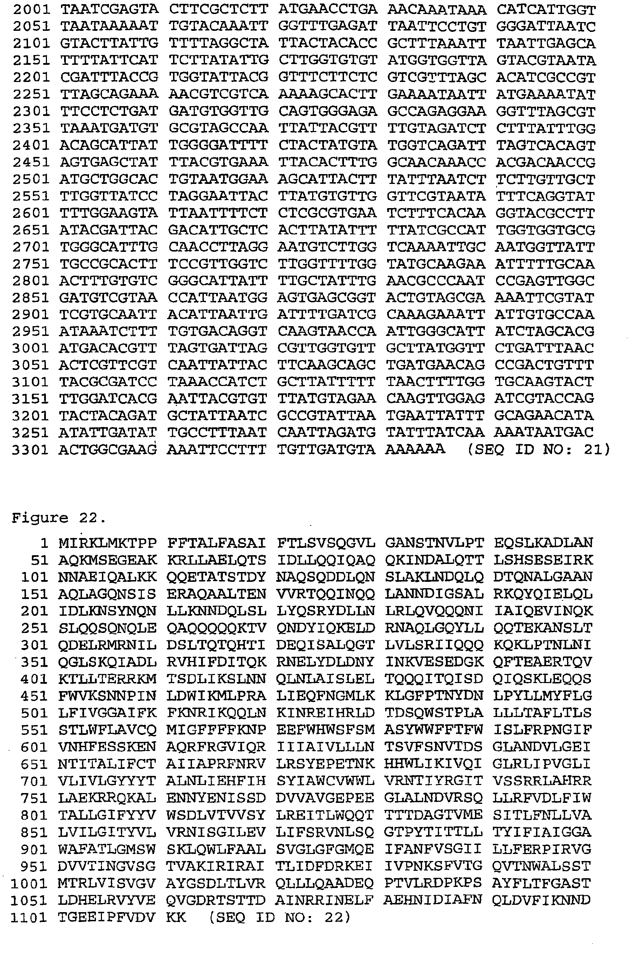

Figure 22 represents the deduced amino acid sequence of the full-length BVH-NTHI11 from nontypeable H. influenzae strain 12085; SEQ ID NO: 22.
Figure 23 represents the DNA sequence of BVH-NTHI12 gene from nontypeable H. influenzae strain 12085; SEQ ID NO: 23
Figure 24 represents the deduced amino acid sequence of the full-length BVH-NTHI12 from nontypeable H. influenzae strain 12085; *SEQ ID NO: 24.*
Figure 25 depicts the comparison of the predicted amino acid sequences of the BVH-NTHI1 open reading frames from 12085, 10095, A18, and A108 H. influenzae by using the program Clustal W from MacVector sequence analysis software (version 6.5). Underneath the alignment, there is a consensus line where (*) characters represent identical amino acid residues and (.) represent conserved amino acid residues.

### DETAILED DESCRIPTION OF THE INVENTION

The present invention provides purified and isolated polynucleotides, which encode H. influenzae polypeptides which may be used to prevent, treat, and/or diagnose H. influenzae infection. The present invention provides twelve separate preferred polynucleotides, each individually and separately defined by one SEQ ID NOs 1, 3, 5, 7, 9, 11, 13, 15, 17, 19, 21 and 23. Further provided in the present invention are twelve separate polypeptides, each individually and separately defined by one of seq ID NOS : 2, 4, 6, 8, 10, 12, 14, 16, 18, 20, 22 and 24. Those skilled in the art will appreciate that the invention includes polynucleotides that encode analogs such as mutants, variants, homologues and derivatives of such polypeptides, as described herein in the present patent application. The invention also includes RNA molecules corresponding to the DNA molecules of the invention. In addition to the DNA and RNA molecules, the invention includes the corresponding polypeptides and monospecific antibodies that specifically bind to such polypeptides.

According to one aspect, the.present invention provides an isolated polynucleotide encoding a polypeptide having at least 70% identity to a second polypeptide comprising a sequence chosen from SEQ ID Nos : 2, 4, 6, 8, 10, 12, 14, 16, 18, 20, 22 and 24 or fragments or analogs thereof.

According to one aspect, the present invention provides an isolated polynucleotide encoding a polypeptide having at least 80% identity to a second polypeptide comprising a sequence chosen from SEQ ID Nos : 2, 4, 6, 8, 10, 12, 14, 16, 18, 20, 22 and 24 or fragments or analogs thereof.

According to one aspect, the present invention provides an isolated polynucleotide encoding a polypeptide having at least 85% identity to a second polypeptide comprising a sequence chosen from SEQ ID Nos : 2, 4, 6, 8, 10, 12, 14, 16, 18, 20, 22 and 24 or fragments or analogs thereof.

According to one aspect, the present invention provides an isolated polynucleotide encoding a polypeptide having at least 90% identity to a second polypeptide comprising a sequence chosen from SEQ ID Nos : 2, 4, 6, 8, 10, 12, 14, 16, 18, 20, 22 and 24 or fragments or analogs thereof.

According to one aspect, the present invention provides an isolated polynucleotide encoding a polypeptide having at least 95% identity to a second polypeptide comprising a sequence chosen from SEQ ID Nos : 2, 4, 6, 8, 10, 12, 14, 16, 18, 20, 22 and 24 or fragments or analogs thereof.

According to one aspect, the present invention provides an isolated polynucleotide encoding a polypeptide comprising a sequence chosen from: SEQ ID NOs: 2, 4, 6, 8, 10, 12, 14, 16, 18, 20, 22 and 24 or fragments or analogs thereof;

According to one aspect, the present relates to polynucleotides encoding an epitope bearing portion of a polypeptide having a sequence chosen from SEQ ID NOs: 2, 4, 6, 8, 10, 12, 14, 16, 18, 20, 22 and 24 or fragments or analogs thereof.

According to one aspect, the present relates to polynucleotides encoding an epitope bearing portion of a polypeptide having a sequence chosen from SEQ ID NOs: 2, 4, 6, 8, 10, 12, 14, 16, 18, 20, 22 and 24.

According to one aspect, the present invention relates to epitope bearing portions of a polypeptide having a sequence chosen from SEQ ID NOs: 2, 4, 6, 8, 10, 12, 14, 16, 18, 20, 22 and 24 or fragments or analogs thereof.

According to one aspect, the present invention relates to epitope bearing portions of a polypeptide having a sequence chosen from SEQ ID NOs: 2, 4, 6, 8, 10, 12, 14, 16, 18, 20, 22 and 24.

According to one aspect, the present invention provides an isolated polynucleotide encoding a polypeptide having at least 70% identity to a second polypeptide comprising a sequence chosen from SEQ ID Nos : 2, 4, 6, 8, 10, 12, 14, 16, 18, 20, 22 and 24.

According to one aspect, the present invention provides an isolated polynucleotide encoding a polypeptide having at least 80% identity to a second polypeptide comprising a sequence chosen from SEQ ID Nos : 2, 4, 6, 8, 10, 12, 14, 16, 18, 20, 22 and 24.

According to one aspect, the present invention provides an isolated polynucleotide encoding a polypeptide having at least 85% identity to a second polypeptide comprising a sequence chosen from SEQ ID Nos : 2, 4, 6, 8, 10, 12, 14, 16, 18, 20, 22 and 24.

According to one aspect, the present invention provides an isolated polynucleotide encoding a polypeptide having at least 90% identity to a second polypeptide comprising a sequence chosen from SEQ ID Nos : 2, 4, 6, 8, 10, 12, 14, 16, 18, 20, 22 and 24.

According to one aspect, the present invention provides an isolated polynucleotide encoding a polypeptide having at least 95% identity to a second polypeptide comprising a sequence chosen from SEQ ID Nos : 2, 4, 6, 8, 10, 12, 14, 16, 18, 20, 22 and 24.

According to one aspect, the present invention provides an isolated polynucleotide encoding a polypeptide comprising a sequence chosen from: SEQ ID NOs: 2, 4, 6, 8, 10, 12, 14, 16, 18, 20, 22 and 24;

According to one aspect, the present relates to polynucleotides encoding an epitope bearing portion of a polypeptide having a sequence chosen from SEQ ID NOs: 2, 4, 6, 8, 10, 12, 14, 16, 18, 20, 22 and 24.

In a further embodiment, the present invention also relates to polynucleotides encoding a polypeptide capable of generating antibodies having binding specificity for a polypeptide having a sequence chosen from SEQ ID NOs: 2, 4, 6, 8, 10, 12, 14, 16, 18, 20, 22 and 24 or fragments or analogs thereof.

In a further embodiment, the present invention also relates to polynucleotides encoding a polypeptide capable of generating antibodies having binding specificity for a polypeptide having a sequence chosen from SEQ ID NOs: 2, 4, 6, 8, 10, 12, 14, 16, 18, 20, 22 and 24.

The percentage of homology is defined as the sum of the percentage of identity plus the percentage of similarity or conservation of amino acid type.

One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of identity analysis are contemplated in the present invention.

In a further embodiment, the polypeptides in accordance with the present invention are antigenic.

In a further embodiment, the polypeptides in accordance with the present invention are immunogenic.

In a further embodiment, the polypeptides in accordance with the present invention can elicit an immune response in an individual.

In a further embodiment, the present invention also relates to polypeptides which are able to raise antibodies having binding specificity to the polypeptides of the present invention as defined above.

In a further embodiment, the present invention also relates to polypeptides capable of generating antibodies having binding specificity for a polypeptide having a sequence chosen from SEQ ID NOs: 2, 4, 6, 8, 10, 12, 14, 16, 18, 20, 22 and 24 or fragments or analogs thereof.

In a further embodiment, the present invention also relates to polypeptides capable of generating antibodies having binding specificity for a polypeptide having a sequence chosen from SEQ ID NOs: 2, 4, 6, 8, 10, 12, 14, 16, 18, 20, 22 and 24.

An antibody that "has binding specificity" is an antibody that recognises and binds the selected polypeptide but which does not substantially recognise and bind other molecules in a sample, e.g., a biological sample. Specific binding can be measured using an ELISA assay in which the selected polypeptide is used as an antigen.

In accordance with the present invention, "protection" in the biological studies is defined by a significant increase in the survival curve, rate or period. Statistical analysis using the Log rank test to compare survival curves, and Fisher exact test to compare survival rates and numbers of days to death, respectively, might be useful to calculate P values and determine whether the difference between the two groups is statistically significant. P values of 0.05 are regarded as not significant.

In an additional aspect of the invention there are provided antigenic/immunogenic fragments of the polypeptides of the invention, or of analogs thereof.

The fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a polypeptide or analog thereof as described herein. The present invention further provides fragments having at least 10 contiguous amino acid residues from the polypeptide sequences of the present invention. In one embodiment, at least 15 contiguous amino acid residues. In one embodiment, at least 20 contiguous amino acid residues.

The key issue, once again, is that the fragment retains the antigenic/immunogenic properties.

The skilled person will appreciate that fragments, analogs or derivatives of the polypeptides of the invention will also find use in the context of the present invention, i.e. as antigenic/immunogenic material. Thus, for instance polypeptides which include one or more additions, deletions, substitutions or the like are encompassed by the present invention.

As used herein, "fragments", "analogs" or "derivatives" of the polypeptides of the invention include those polypeptides in which one or more of the amino acid residues are substituted with a conserved amino acid residue (preferably conserved) and which may be natural or unnatural. In one embodiment, derivatives and analogs of polypeptides of the invention will have about 70% identity with those sequences illustrated in the figures or fragments thereof. That is, 70% of the residues are the same. In a further embodiment, polypeptides will have greater than 80% identity. In a further embodiment, polypeptides will have greater than 90% identity. In a further embodiment, polypeptides will have greater than 95% identity. In a further embodiment, polypeptides will have greater than 99% identity. In a further embodiment, analogs of polypeptides of the invention will have fewer than about 20 amino acid residue substitutions, modifications or deletions and more preferably less than 10.

In one embodiment, derivatives and analogs of polypeptides of the invention will have about 70% homology with those sequences illustrated in the figures or fragments thereof. In a further embodiment, derivatives and analogs of polypeptides will have greater than 80% homology. In a further embodiment, derivatives and analogs of polypeptides will have greater than 90% homology. In a further embodiment, derivatives and analogs of polypeptides will have greater than 95% homology. In a further embodiment, derivatives and analogs of polypeptides will have greater than 99% homology. In a further embodiment, derivatives and analogs of derivatives and analogs of polypeptides of the invention will have fewer than about 20 amino acid residue substitutions, modifications or deletions and more preferably less than 10.

According to a further aspect, the invention provides polypeptides having at least 70% identity to a second polypeptide having an amino acid sequence chosen from: SEQ ID NOs: .2, 4, 6, 8, 10, 12, 14, 16, 18, 20, 22 and 24 or fragments or analogs thereof.

According to a further aspect, the invention provides polypeptides having at least 80% identity to a second polypeptide having an amino acid sequence chosen from: SEQ ID NOs: 2, 4, 6, 8, 10, 12, 14, 16, 18, 20, 22 and 24 or fragments or analogs thereof.

According to a further aspect, the invention provides polypeptides having at least 85% identity to a second polypeptide having an amino acid sequence chosen from: SEQ ID NOs: 2, 4, 6, 8, 10, 12, 14, 16, 18, 20, 22 and 24 or fragments or analogs thereof.

According to a further aspect, the invention provides polypeptides having at least 90% identity to a second polypeptide having an amino acid sequence chosen from: SEQ ID NOs: 2, 4, 6, 8, 10, 12, 14, 16, 18, 20, 22 and 24 or fragments or analogs thereof.

According to a further aspect, the invention provides polypeptides having at least 95% identity to a second polypeptide having an amino acid sequence chosen from: SEQ ID NOs: 2, 4, 6, 8, 10, 12, 14, 16, 18, 20, 22 and 24 or fragments or analogs thereof.

According to a further aspect, the invention provides polypeptides comprising a sequence chosen from: SEQ ID NOs: 2, 4, 6, 8, 10, 12, 14, 16, 18, 20, 22 and 24 or fragments or analogs thereof.

According to a further aspect, the invention provides polypeptides characterized by a sequence chosen from: SEQ ID NOs: 2, 4, 6, 8, 10, 12, 14, 16, 18, 20, 22 and 24 or fragments or analogs thereof.

According to a further aspect, the invention provides polypeptides having at least 70% identity to a second polypeptide having an amino acid sequence chosen from: SEQ ID NOs: 2, 4, 6, 8, 10, 12, 14, 16, 18, 20, 22 and 24.

According to a further aspect, the invention provides polypeptides having at least 80% identity to a second polypeptide having an amino acid sequence chosen from: SEQ ID NOs: 2, 4, 6, 8, 10, 12, 14, 16, 18, 20, 22 and 24.

According to a further aspect, the invention provides polypeptides having at least 85% identity to a second polypeptide having an amino acid sequence chosen from: SEQ ID NOs: 2, 4, 6, 8, 10, 12, 14, 16, 18, 20, 22 and 24.

According to a further aspect, the invention provides polypeptides having at least 90% identity to a second polypeptide having an amino acid sequence chosen from: SEQ ID NOs: 2, 4, 6, 8, 10, 12, 14, 16, 18, 20, 22 and 24.

According to a further aspect, the invention provides polypeptides having at least 95% identity to a second polypeptide having an amino acid sequence chosen from: SEQ ID NOs: 2, 4, 6, 8, 10, 12, 14, 16, 18, 20, 22 and 24.

According to a further aspect, the invention provides polypeptides comprising a sequence chosen from: SEQ ID NOs: 2, 4, 6, 8, 10, 12, 14, 16, 18, 20, 22 and 24.

According to a further aspect, the invention provides polypeptides characterized by a sequence chosen from: SEQ ID NOs: 2, 4, 6, 8, 10, 12, 14, 16, 18, 20, 22 and 24.

These substitutions are those having a minimal influence on the secondary structure and hydropathic nature of the polypeptide. Preferred substitutions are those known in the art as conserved, i.e. the substituted residues share physical or chemical properties such as hydrophobicity, size, charge or functional groups. These include substitutions such as those described by Dayhoff, M. in Atlas of Protein Sequence and Structure 5, 1978 and by Argos, P. in EMBO J. 8, 779-785, 1989. For example, amino acids, either natural or unnatural, belonging to one of the following groups represent conservative changes:
ala, pro, gly, gln, asn, ser, thr, val;
cys, ser, tyr, thr;
val, ile, leu, met, ala, phe;
lys, arg, orn, his;
and phe, tyr, trp, his.

The preferred substitutions also include substitutions of D-enantiomers for the corresponding L-amino acids.

Preferably, a fragment, analog or derivative of a polypeptide of the invention will comprise at least one antigenic region i.e. at least one epitope.

In an alternative approach, the analogs could be fusion polypeptides, incorporating moieties which render purification easier, for example by effectively tagging the desired polypeptide. It may be necessary to remove the "tag" or it may be the case that the fusion polypeptide itself retains sufficient antigenicity to be useful.

Thus, what is important for analogs, derivatives and fragments is that they possess at least a degree of the antigenicity/immunogenic of the protein or polypeptide from which they are derived.

Also included are polypeptides which have fused thereto other compounds which alter the biological or pharmacological properties of the polypeptide, i.e., polyethylene glycol (PEG) to increase half-life, leader or secretory amino acid sequences for ease of purification, prepro- and pro- sequences and (poly)saccharides.

Furthermore, in those situations where amino acid regions are found to be polymorphic, it may be desirable to vary one or more particular amino acids to more effectively mimic the different epitopes of the different H. influenzae strains.

Moreover, the polypeptides of the present invention can be modified by terminal -NH₂ acylation (e.g. by acetylation or thioglycolic acid amidation, terminal carboxy amidation, e.g. with ammonia or methylamine) to provide stability, increased hydrophobicity for linking or binding to a support or other molecule.

Also contemplated are hetero and homo polypeptide multimers of the polypeptide fragments and analogues. These polymeric forms include, for example, one or more polypeptides that have been cross-linked with cross-linkers such as avidin/biotin, glutaraldehyde or dimethylsuperimidate. Such polymeric forms also include polypeptides containing two or more tandem or inverted contiguous sequences, produced from multicistronic mRNAs generated by recombinant DNA technology.

In a further embodiment, the present invention also relates to chimeric polypeptides which comprise one or more polypeptides or fragments or analogs or derivatives thereof as defined in the figures of the present application.

In a further embodiment, the present invention also relates to chimeric polypeptides comprising two or more polypeptides having a sequence chosen from SEQ ID NOs: 2, 4, 6, 8, 10, 12, 14, 16, 18, 20, 22 and 24 or fragments or analogs thereof; provided that the polypeptides are linked as to formed a chimeric polypeptide.

In a further embodiment, the present invention also relates to chimeric polypeptides comprising two or more polypeptides having a sequence chosen from SEQ ID NOs : 2, 4, 6, 8, 10, 12, 14, 16, 18, 20, 22 and 24; provided that the polypeptides are linked as to formed a chimeric polypeptide.

In order to achieve the formation of antigenic polymers (i.e. synthetic multimers), polypeptides may be utilized having bishaloacetyl groups, nitroarylhalides, or the like, where the reagents being specific for thio groups. Therefore, the link between two mercapto groups of the different peptides may be a single bound or may be composed of a linking group of at least two, typically at least four and not more than 16, but usually not more than about 14 carbon atoms.

In a particular embodiment, polypeptide fragments or analogs of the invention do not contain a methionine (Met) starting residue. Preferably, polypeptides will not incorporate a leader or secretory sequence (signal sequence). The signal portion of a polypeptide of the invention may be determined according to established molecular biological techniques. In general, the polypeptide of interest may be isolated from Haemophilus culture and subsequently sequenced to determine the initial residue of the mature protein and therefore the sequence of the mature polypeptide.

The following Table A describes the signal sequences of the polypeptides of the invention as described in the Figures.

| Polypeptide | SEQ ID No | Secretion signal |
|---|---|---|
| BVH-NTHI1 | 2 | MPVIRQVVFYDSLTGEQTKMKKFAGLITASFVA |
| BVH-NTHI2 | 4 | MKKLLKISAVSAALLSAPMMA |
| BVH-NTHI3 | 6 | MLMKLKATLTLAAATLVLAA |
| BVH-NTHI4 | 8 | MMNRRHFIQIGATSILALSANRFAMA |
| BVH-NTHI5 | 10 | MKKIILTLSLGLLTAWSAQI |
| BVH-NTHI6 | 12 | no secretion signal |
| BVH-NTHI7 | 14 | MKKFLIAILLLILILAGAA |
| BVH-NTHI8 | 16 | MKMKKFILKSFLLATLGCVAFASMAQA |
| BVH-NTHI9 | 18 | MTMFKKISVLFFTLILAGCSSWS |
| BVH-NTHI10 | 20 | MSILLQGERFKKRLMPILLSMALAGCSNLLG |
| BVH-NTHI11 | 22 | MIRKLMKTPPFFTALFASAIFTLSVSQGVLG |
| BVH-NTHI12 | 24 | MKLKQLFAITAIA |

According to another aspect of the invention, there are also provided (i) a composition of matter containing a polypeptide of the invention, together with a carrier, diluent or adjuvant; (ii) a pharmaceutical composition comprising a polypeptide of the invention and a carrier, diluent or adjuvant; (iii) a vaccine comprising a polypeptide of the invention and a carrier, diluent or adjuvant; (iv) a method for inducing an immune response against Haemophilus, in an individual, by administering to the individual, an immunogenically effective amount of a polypeptide of the invention to elicit an immune response, e.g., a protective immune response to Haemophilus; and particularly, (v) a method for preventing and/or treating a Haemophilus infection, by administering a prophylactic or therapeutic amount of a polypeptide of the invention to an individual in need.

Before immunization, the polypeptides of the invention can also be coupled or conjugated to carrier proteins such as tetanus toxin, diphtheria toxin, hepatitis B virus surface antigen, poliomyelitis virus VP1 antigen or any other viral or bacterial toxin or antigen or any suitable proteins to stimulate the development of a stronger immune response. This coupling or conjugation can be done chemically or genetically. A more detailed description of peptide-carrier conjugation is available in Van Regenmortel, M.H.V., Briand J.P., Muller S., Plaué S., «Synthetic Polypeptides as antigens» in Laboratory Techniques in Biochemistry and Molecular Biology, Vol.19 (ed.) Burdou, R.H. & Van Knippenberg P.H. (1988), Elsevier New York.

According to another aspect, there are also provided pharmaceutical compositions comprising one or more Haemophilus polypeptides of the invention in a mixture with a pharmaceutically acceptable carrier, diluent or adjuvant. Suitable adjuvants include (1) oil-in-water emulsion formulations such as MF59^{™}, SAF^{™}, Ribi^{™} ; (2) Freund's complete or incomplete adjuvant; (3) salts i.e. AlK (SO₄)₂, AlNa (SO₄)₂, AlNH₄(SO₄)₂, Al (OH)₃, AlPO₄, silica, kaolin; (4) saponin derivatives such as stimulon™ or particles generated therefrom such as ISCOMs (immunostimulating complexes); (5) cytokines such as interleukins, interferons, macrophage colony stimulating factor (M-CSF), tumor necrosis factor (TNF) ; (6) other substances such as carbon polynucleotides i.e. poly IC and poly AU, detoxified cholera toxin (CTB)and E.coli heat labile toxin for induction of mucosal immunity. A more detailed description of adjuvant is available in a review by M.Z.I Khan et al. in Pharmaceutical Research, vol. 11, No. 1 (1994) pp2-11, and also in another review by Gupta et al., in Vaccine, Vol. 13, No. 14, pp1263-1276 (1995) and in WO 99/24578. Preferred adjuvants include QuilA™, QS21™, Alhydrogel™ and Adjuphos™.

Pharmaceutical compositions of the invention may be administered parenterally by injection, rapid infusion, nasopharyngeal absorption, dermoabsorption, or buccal or oral.

Pharmaceutical compositions of the invention are used for the treatment or prophylaxis of Haemophilus infection and/or diseases and symptoms mediated by Haemophilus infection as described in P.R. Murray (Ed, in chief),E.J. Baron, M.A. Pfaller, F.C. Tenover and R.H. Yolken. Manual of Clinical Microbiology, ASM Press, Washington, D.C. seventh edition, 1999, p1481-1482 which are herein incorporated by reference. In one embodiment, pharmaceutical compositions of the present invention are used for the treatment or prophylaxis of otitis media (acute or recurrent), sinusitis, bronchitis, pneumonia, meningitis and bacteremia. In one embodiment, pharmaceutical compositions of the invention are used for the treatment or prophylaxis of Haemophilus infection and/or diseases and symptoms mediated by Haemophilus infection. In a further embodiment, the Haemophilus infection is Haemophilus Influenzae. In a further embodiment, the Haemophilus infection is Nontypeable Haemophilus Influenzae. In a further embodiment, the Haemophilus infection is Typeable Haemophilus Influenzae.

As used in the present application, the term "individual" include mammal. In a further embodiment, the mammal is human.

In a particular embodiment, pharmaceutical compositions are administered to those individuals at risk of H. influenzae infection such as infants, elderly and immunocompromised individuals.

Pharmaceutical compositions are preferably in unit dosage form of about 0.001 to 100 µg/kg (antigen/body weight) and more preferably 0.01 to 10 µg/kg and most preferably 0.1 to 1 µg/kg 1 to 3 times with an interval of about 1 to 6 week intervals between immunizations.

Pharmaceutical compositions are preferably in unit dosage form of about 0.1 µg to 10 mg and more preferably 1µg to 1 mg and most preferably 10 to 100 µg 1 to 3 times with an interval of about 1 to 6 week intervals between immunizations.

In one embodiment, polynucleotides are those illustrated in SEQ ID Nos : 1, 3, 5, 7, 9, 11, 13, 15, 17, 19, 21 and 23 which may include the open reading frames (ORF), encoding the polypeptides of the invention.

In a further embodiment, polynucleotides are those illustrated in SEQ ID NO: 1, 3, 5, 7, 9, 11, 13, 15, 17, 19, 21, 23 encoding polypeptides of the invention.

It will be appreciated that the polynucleotide sequences illustrated in the figures may be altered with degenerated codons yet still encode the polypeptide of the invention. Accordingly the present invention further provides polynucleotide herein above described (or the complement sequence thereof) having 50% identity between sequences. In one embodiment, at least 70% identity between sequences. In one embodiment, at least 75% identity between sequences. In one embodiment, at least 80% identity between sequences. In one embodiment, at least 85% identity between sequences. In one embodiment, at least 90% identity between sequences. In a further embodiment, polynucleotides are hybridizable under stringent conditions, i.e. having at least 95% identity. In a further embodiment, more than 97% identity.

Suitable stringent conditions for hybridation can be readily determined by one of skilled in the art (see for example Sambrook et al., (1989) Molecular cloning : A Laboratory Manual, 2^{nd} ed, Cold Spring Harbor, N.Y.; Current Protocols in Molecular Biology, (1999) Edited by Ausubel F.M. et al., John Wiley & Sons, Inc., N.Y.).

In a further embodiment, the present invention provides polynucleotides that hybridize under stringent conditions to either
(a) a DNA sequence encoding a polypeptide or
(b) the complement of a DNA sequence encoding a polypeptide; wherein said polypeptide comprises SEQ ID NO: 2, 4, 6, 8, 10, 12, 14, 16, 18, 20, 22, 24, or fragments or analogs thereof.

In a further embodiment, the present invention provides polynucleotides that hybridize under stringent conditions to either
(a) a DNA sequence encoding a polypeptide or
(b) the complement of a DNA sequence encoding a polypeptide; wherein said polypeptide comprises at least 10 contiguous amino acid residues from a polypeptide comprising SEQ ID NO: 2, 4, 6, 8, 10, 12, 14, 16, 18, 20, 22, 24 or fragments or analogs thereof.

In a further embodiment, the present invention provides polynucleotides that hybridize under stringent conditions to either
(a) a DNA sequence encoding a polypeptide or
(b) the complement of a DNA sequence encoding a polypeptide; wherein said polypeptide comprises SEQ ID NO: 2, 4, 6, 8, 10, 12, 14, 16, 18, 20, 22, 24.

In a further embodiment, the present invention provides polynucleotides that hybridize under stringent conditions to either
(a) a DNA sequence encoding a polypeptide or
(b) the complement of a DNA sequence encoding a polypeptide; wherein said polypeptide comprises at least 10 contiguous amino acid residues from a polypeptide comprising SEQ ID NO: 2, 4, 6, 8, 10, 12, 14, 16, 18, 20, 22, 24.

As will be readily appreciated by one skilled in the art, polynucleotides include both DNA and RNA.

The present invention also includes polynucleotides complementary to the polynucleotide described in the present application.

In a further aspect, polynucleotides encoding polypeptides of the invention, or fragments or analogs thereof, may be used in a DNA immunization method. That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably, the vector is injected intramuscularly.

According to another aspect, there is provided a process or method of manufacturing for producing polypeptides of the invention by recombinant techniques by expressing a polynucleotide encoding said polypeptide in a host cell and recovering the expressed polypeptide product. Alternatively, the polypeptides can be produced according to established synthetic chemical techniques, i.e. solution phase or solid phase synthesis of oligopeptides which are ligated to produce the full polypeptide (block ligation).

General methods for obtention and evaluation of polynucleotides and polypeptides are described in the following references : Sambrook et al., (1989) Molecular cloning : A Laboratory Manual, 2nd ed, Cold Spring Harbor, N.Y.; Current Protocols in Molecular Biology, (1999) Edited by Ausubel F.M. et al., John Wiley & Sons, Inc., N.Y.; PCR Cloning Protocols, from Molecular Cloning to Genetic Engineering, (1997) Edited by white B.A., Humana Press, Totowa, New Jersey, 490 pages; Protein Purification, Principles and Practices, (1993) Scopes R.K., Springer-Verlag, N.Y., 3rd Edition, 380 pages; Current Protocols in Immunology, (1999) Edited by Coligan J.E. et al., John Wiley & Sons Inc., N.Y., are herein incorporated by reference.

For recombinant production, host cells are transfected with vectors which encode the polypeptides of the invention, and then cultured in a nutrient media modified as appropriate for activating promoters, selecting transformants or amplifying the genes. Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook et al., (1989) Molecular Cloning: A Laboratory manual, 2nd ed., Cold Spring Harbor, N.Y.; Current Protocols in Molecular Biology, (1999) Edited by Ausubel F.M. et al., John Wikey & Sons, Inc., N.Y., incorporated herein by reference). Suitable promoters include but are not limited to LTR or SV40 promoter, E.coli lac, tac or trp promoters and the phage lambda P_{L} promoter. Vectors will preferably incorporate an origin of replication as well as selection markers i.e. ampicilin resistance gene. Suitable bacterial vectors include pET, pQE70, pQE60, pQE-9, pD10 phagescript, psiX174, pbluescript SK, pbsks, pH8A, pNH16a, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial i.e. E.coli, Bacillus subtilis, Streptomyces; fungal i.e. Aspergillus niger, Aspergillus nidulins; yeast i.e. Saccharomyces or eukaryotic i.e. CHO, COS.

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptidei.e. using ammonium sulfate or ethanol precipitation, acid extraction, anion or cation exchange chromatography, phosphocellulose chromatography hydrophobic interaction chromatography, hydroxylapatite chromatography and lectin chromatography. Final purification may be achieved using HPLC.

The polypeptide may be expressed with or without a leader or secretion sequence. In the former case, the leader may be removed using post-translational processing (see US. 4 431 739, US 4 425 437 and US 4 338 397 incorporated herein by reference) or be chemically removed subsequent to purifying the expressed polypeptide.

According to a further aspect, the Haemophilus polypeptides of the invention may be used in a diagnostic test for H. influenzae infection, in particular for H. influenzae infection. Several diagnostic methods are possible, for example detecting Haemophilus organismn in a biological sample, the following procedure may be followed :
a. obtaining a biological sample from an individual;
b. incubating an antibody or fragment thereof reactive with an Haemophilus polypeptide of the invention with the biological sample to form a mixture, and
c. detecting specifically bound antibody or bound fragment in the mixture which indicates the presence of Haemophilus.

Alternatively, a method for the detection of antibody specific to an H. influenzae antigen in a biological sample containing or suspected of containing said antibody may be performed as follows :
a. obtaining a biological sample from an individual;
b. incubating one or more H. influenzae polypeptides of the invention or fragments thereof with the biological sample to form a mixture; and
c. detecting specifically bound antigen or bound fragment in the mixture which indicates the presence of antibody specific to H. influenzae.

One of skill in the art will recognize that this diagnostic test may take several forms, including an immunological test such as an enzyme-linked immunoadsorbent assay (ELISA), a radioimmunoassay or a latex agglutination assay, essentially to determine whether antibodies specific for the polypeptides are present in an organism.

According to a further aspect, the invention relates to a method for prophylactic or therapeutic treatment of otitis media, sinusitis, bronchitis, pneumonia and meningitis and bacteremia comprising administering to an individual a therapeutic or prophylactic amount of a composition of the invention.

According to a further aspect, the invention relates to a method for prophylactic or therapeutic treatment of Haemophilus influenzae bacterial infection in an individual susceptible to Haemophilus influenzae infection comprising administering to said individual a therapeutic or prophylactic amount of a composition of the invention. In a further embodiment, Haemophilus influenzae is Nontypeable Haemophilus influenzae. In a further embodiment, Haemophilus influenzae is Typeable Haemophilus influenzae.

According to a further aspect, the invention relates to a method for diagnostic of otitis media, sinusitis, bronchitis, pneumonia and meningitis and bacteremia comprising administering to an individual a therapeutic or prophylactic amount of a composition of the invention.

According to a further aspect, the invention relates to a method for diagnostic of Haemophilus influenzae bacterial infection in an individual susceptible to Haemophilus influenzae infection comprising administering to said individual a therapeutic or prophylactic amount of a composition of the invention. In a further embodiment, Haemophilus influenzae is Nontypeable Haemophilus influenzae. In a further embodiment, Haemophilus influenzae is Typeable Haemophilus influenzae.

According to a further aspect, the invention relates to the use of a pharmaceutical composition of the invention for the prophylactic or therapeutic treatment of Haemophilus infection comprising administering to said individual a prophylactic or therapeutic amount of the composition.

According to a further aspect, the Haemophilus polypeptides of the invention may be used in a kit comprising the polypeptides of the invention for detection of diagnosis of Hameophilus infection.

The DNA sequences encoding polypeptides of the invention may also be used to design DNA probes for use in detecting the presence of H. influenzae in a biological sample suspected of containing such bacteria. The detection method of this invention comprises :
a. obtaining the biological sample from an individual;
b.incubating one or more DNA probes having a DNA sequence encoding a polypeptide of the invention or fragments thereof with the biological sample to form a mixture; and
c.detecting specifically bound DNA probe in the mixture which indicates the presence of H. influenzae bacteria.

The DNA probes of this invention may also be used for detecting circulating H. influenzae (i.e. H. influenzae nucleic acids) in a sample, for example using a polymerase chain reaction, as a method of diagnosing H. influenzae infections. The probe may be synthesized using conventional techniques and may be immobilized on a solid phase or may be labelled with a detectable label. A preferred DNA probe for this application is an oligomer having a sequence complementary to at least about 6 contiguous nucleotides of the H. influenzae polypeptides of the invention.

Another diagnostic method for the detection of H. influenzae in an individual comprises :
a. labelling an antibody reactive with a polypeptide of the invention or fragment thereof with a detectable label;
b. administering the labelled antibody or labelled fragment to the individual; and
c. detecting specifically bound labelled antibody or labelled fragment in the individual which indicates the presence of H. influenzae.

A further aspect of the invention is the use of the Haemophilus polypeptides of the invention as immunogens for the production of specific antibodies for the diagnosis and in particular the treatment of H. influenzae infection. Suitable antibodies may be determined using appropriate screening methods, for example by measuring the ability of a particular antibody to passively protect against H. influenzae infection in a test model. One example of an animal model is the mouse model described in the example herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal or preferably monoclonal. It may be specific for a number of epitopes associated with the H. influenzae polypeptides but is preferably specific for one.

A further aspect of the invention is the use of a pharmaceutical composition of the invention for the prophylactic or therapeutic treatment of Haemophilus infection comprising administering to said individual a prophylactic or therapeutic amount of the composition.

Unless otherwise defined, all technical and scientific terms used herein have the same meaning as commonly understood by one of ordinary skill in the art to which this invention belong.

### ISOLATION OF NONTYPEABLE H. INFLUENZAE (NTHI) POLYPEPTIDES AND IDENTIFICATION OF GENES ENCODING THESE POLYPEPTIDES

### ISOLATION OF SURFACE-EXPOSED NTHI POLYPEPTIDES

### Bacterial strains

Nontypeable H. influenzae clinical isolates 12085 and 10095 were provided by D.M. Granoff (St-Louis, Missouri) and B-20, A18, A108, C-98, C-26 and B-31 by the Centre de Recherche en Infectiologie du Centre Hospitalier de l'Université Laval. Nontypeable H. influenzae 12085 was isolated from the blood of a child with lower respiratory tract infection in Pakistan.

Escherichia coli DH5α. (GIBCO BRL, Burlington, Ontario) was used as the host strain for recombinant DNA. E. coli XL1-Blue MRF' (Stratagene, LaJolla, California) was used as the host strain for infection with lambda ZAP Express phage vector. E. coli XLOLR (Stratagene) was used as the host strain for infection with in vivo excised filamentous lambda ZAP Express.

### Antisera

Mice antisera was produced following three-week intervals subcutaneous immunization with outer membrane proteins in presence of complete or incomplete Freund adjuvants (Cedarlane Laboratories Ltd, Hornby, Canada). Sera was collected 21 days after the tertiary immunization.

Four human antisera were selected on the basis of their reactivity by ELISA and Western blotting on heat-killed nontypeable H. influenzae 12085.

### H. influenzae library construction and screening

Nontypeable H. influenzae 12085 DNA was extracted using a genomic extraction Kit (QIAgen). DNA was partially digested with Tsp509I and ligated to EcoRI digested λ-ZAP Express phage arms (Stratagene). The ligation was packaged in vitro with Gigapack extracts according to the manufacturer's recommendations (Stratagene). Recombinant phage were plated on E. coli XL1-Blue MRF' at density of 2.5 x 10⁴ PFU/150 mm (diameter) plate. Following eight hours of incubation, the plates were overlaid with nitrocellulose disks and the resulting lifts were processed for immunoblotting with human and mice sera. Positive clones were picked up and plaque-purified. Recombinant pBK-CMV plasmids coding for the Haemophilus genes of interest were recovered from the purified bacteriophage using ExAssist filamentous helper phage and the lambda-resistant strain E. coli XLOLR by in vivo excision.

### Identification of antigens

SDS-PAGE and immunoblot analysis of lysates of these recombinant E. coli showed that each clone expressed one or more polypeptides that reacted with human or mice antisera.

### DNA sequencing and sequence analysis

The genomic clones were sequenced with the Taq DyeDeoxy Terminator Cycle Sequencing Kit (Applied Biosystem, Foster City, California). Several internal primers were designed to sequence further into the cloned inserts. Sequence assembly was performed using Sequencher software (Gene Codes Corporation, Ann Arbor, Michigan) and sequence analysis was performed with McVector software (Oxford Molecular Ltd., Campbell, California).

### EXAMPLE 1

This example illustrates the cloning of nontypeable H. influenzae genes into an expression vector

The coding regions of nontypeable H. influenzae genes BVH-NTHI1 to BVH-NTHI12 (SEQ ID NO:1,3,5,7,9,11,13,15,17,19,21 and 23) were amplified by PCR (DNA Thermal Cycler GeneAmp PCR system 2400 Perkin Elmer, San Jose, CA) from genomic DNA of nontypeable H. influenzae strain 12085 by using the following oligonucleotide primers that contained base extensions for the addition of restriction sites *Nco*I (CCATGG), NdeI (CATATG), *Sal*I (GTCGAC) or XhoI (CTCGAG) (Table 1). PCR products were purified from agarose gels by using a QIAquick gel extraction kit from QIAgen following the manufacturer's instructions (Chatsworth, CA), and digested with appropriate restriction endonucleases (Pharmacia Canada Inc, Baie d'Urfé, Canada). The pET vectors (Novagen, Madison, Wisconsin) were digested likewise and purified from agarose gels using a QIAquick gel extraction kit from QIAgen (Chatsworth, California). The digested PCR products were ligated to the enzyme-restricted pET expression vector. The ligated products were transformed into E. coli strain DH5α [φ80d*lac*ZΔM15 Δ(*lac*ZYA-*arg*F)U169 *end*A1 *rec*A1 *hsd*R17(r_{K}-m_{K}+) *deo*R *thi*-1 *sup*E44 λ-*gyr*A96 *rel*A1] (Gibco BRL, Gaithersburg, Maryland) according to the method of Simanis (Hanahan D. (1985) In : DNA Cloning. D.M. Glover, ed., IRL Press, Washington D.C. vol. 1 pp. 109-135). Recombinant gene-containing plasmids (rpET) were purified using a QIAgen kit (Chatsworth, California) and DNA inserts were sequenced (Taq Dye Deoxy Terminator Cycle Sequencing kit, ABI, Foster City, California).

**Table 1. List of oligonucleotide primers for DNA cloning.**

| PCR Primer set | Seq. ID. | Sequence 5`-3' | Nucleotide position* | Restriction sites | Cloning vector-Strain |
|---|---|---|---|---|---|
| | | | | | |
| HAMJ342- | 25 | CAAGGCGTTTTCATATG CCTGTCATTCGGCAGG | 1-1137 | *Nde*I | pET21-b+ |
| HAMJ343 | 26 | CTAATTGACCTCGAGTT TTGCTGCTTTTAATTCT TGATAATATTG | | *Xho*I | Tuner (DE3) |
| HAMJ345- | 27 | GTAAGGAAACATACATA TGAAAAAACTTTTAAAA ATTAGTG | 1-1005 | *Nde*I | pET21-b+ |
| HAMJ344 | 28 | TTAGAGACTCGAGTTTA GCTAAACATTCTATGTA GCTATC | | *Xho*I | BL21 (DE3) |
| HAMJ348- | 29 | CCTAACATTGACATATG CTTATGAAACTAAAAGC AACA | 1-1647 | *Nde*I | pET21-b+ |
| HAMJ349 | 30 | TCAATATCTCGAGTTTA CCATCAACACTCACACC | | *Xho*I | AD494 (DE3) |
| HAMJ346- | 31 | CTAATAAGGAAAACCAT ATGATGAACAGACGTCA TTTTATTC | 1-1971 | *Nde*I | pET21-b+ |
| HAMJ399 | 32 | GACCTAGCTCGAGTTTA GATAAATCAATTTGATA CACTG | | *Xho*I | Tuner(DE3) |
| HAMJ376- | 33 | CATAAGGAGTAACATAT GAAAAAAATTATTTTAA CATTATC | 1-480 | *Nde*I | pET21-b+ |
| HAMJ377 | 34 | GTGCAGATTCTCGAGTT TTTTATCAACTGAA | | *Xho*I | Tuner (DE3) |
| HAMJ460- | 35 | CTGGACAGACCATATGC CTTCTGATTTAGTCGC | 2-936 | *Nde*I | pET21-b+ |
| HAMJ461 | 36 | CTAAATTGAGCTCGAGA TTAGTTTTTAATTTACT CC | | *XHo*I | Tuner(DE3) |
| HAMJ458- | 37 | CTTTCTTATAGCATATG AAGAAATTTTTAATTGC GATT | 1-1041 | *Nde*I | pET21-b+ |
| HAMJ459 | 38 | CTTCCGCACTCGAGTTT GGCATTTTTC | | *Xho*I | Tuner (DE3)pLysS |
| HAMJ477- | 39 | GGAAGGAGCTAGCATGA AAATGAAAAAATTTATT C | 1-939 | *Nhe*I | pET21-b+ |
| HAMJ478 | 40 | TTGATACTCTCGAGTTT ATTAAAATACTG | | *Xho*I | Tuner(DE3) |
| HAMJ481- | 41 | CTTTAATCACATATGAC TATGTTTAAAAAAATCT CTG | 1-534 | *Nde*I | pET21-b+ |
| HAMJ482 | 42 | CACCAATCTCGAGTTTA GATGTACAGGCTT | | *Xho*I | |
| HAMJ78- | 43 | ACCCGCATGGCTGTTCA AATCTACTTGGTAG | 75-1728 | *Nco*I | pET32-c+ |
| HAMJ79 | 44 | ACTCGTCGACTTAGTTT GCAACTGGTACAAT | | *Sal*I | AD494 (DE3) |
| HAMJ414- | 45 | GATTAGGGAAAACATAT GATTAGGAAACTTATGA A | 1-3336 | *Nde*I | pET21-b+ |
| HAMJ415 | 46 | CCATAATTTGCTCGAGT TTTTTTACATCAAC | | *Xho*I | |
| HAMJ450- | 47 | GGAAGGAGCTAGCATGA AATTAAAACAACTTTTT G | 1-816 | *Nhe*I | pET21-b+ |
| HAMJ411 | 48 | GTGCGGTAGCTCGAGCC AACCTTTTAC | | *Xho*I | Tuner(DE3) |

| | | | | | |
|---|---|---|---|---|---|
| * Nucleotide position indicates the result of the cloning, i.e. the length ant the position in Figure 1 of the PCR products. | | | | | |

### EXAMPLE 2

This example describes the PCR amplification and sequencing of BVH-NTHI1 gene from other nontypeable H. influenzae strains and the evaluation of the level of molecular conservation of this gene.

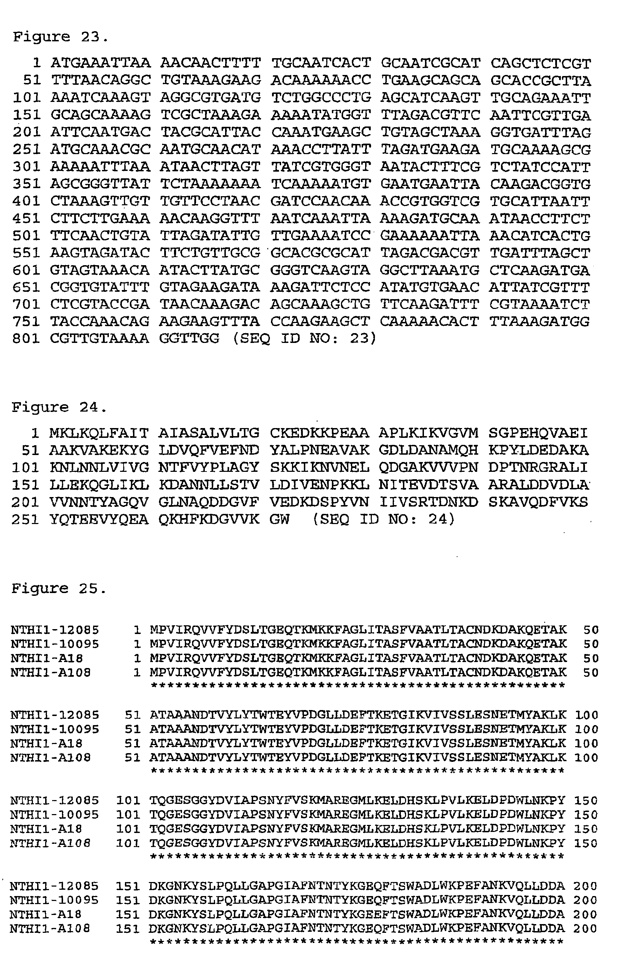

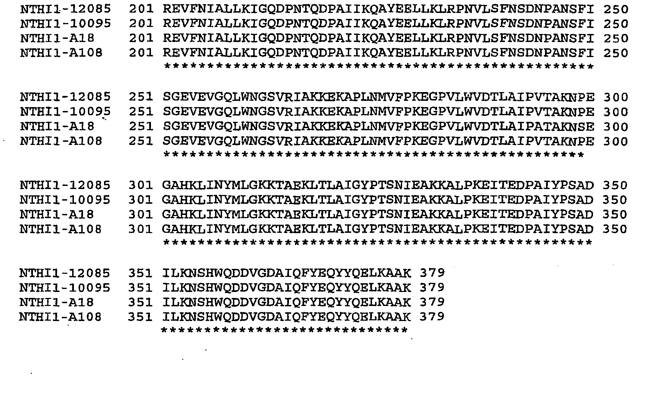

The respective coding regions of nontypeable H. influenzae gene BVH-NTHI1 from these strains were amplified by PCR (DNA Thermal Cycler GeneAmp PCR system 2400 Perkin Elmer, San Jose, CA) from genomic DNA using the following oligos: HAMJ342 (5'-CAAGGCGTTTTCATATGCCTGTCATTCGGCAGG -3'); HAMJ343 (5'-CTAATTGACCTCGAGTTTTGCTGCTTTTAATTCTTGATAATATTG -3'). PCR products were purified from agarose gels using a QIAquick gel extraction kit from QIAgen following the manufacturer's instructions (Chatsworth, CA) and the DNA inserts were sequenced (Taq Dye Deoxy Terminator Cycle Sequencing kit, ABI, Foster City, CA). The length of PCR fragments generated for all these eight strains was identical. Pairwise comparisons of nucleotides and predicted amino acid sequences from four of these strains (12085, 10095, A18 and A108) revealed 99% identity as shown in Figure 25.

### EXAMPLE 3

This example illustrates the production and purification of recombinant nontypeable H. influenzae BVH-NTHI1 polypeptide.

The recombinant pET21-b+ plasmid with BVH-NTHI1 gene corresponding to the SEQ ID NO: 1 was used to electrotransform (Gene Pulser II apparatus, BIO-RAD Labs, Mississauga, Canada) E. coli strain Tuner (DE3) [ F⁻ ompT, hsdS (r_{b}, m_{b}), gal, dcn, lacYI (DE3)] (Novagen, Madison, Wisconsin). Within this E. coli strain, the T7 promotor controlling expression of the recombinant polypeptide is specifically recognized by the T7 RNA polymerase (present on the λDE3 prophage) whose gene is under the control of the lac promotor which is inducible by isopropyl-ß-d-thio-galactopyranoside (IPTG). The transformant Tuner(DE3)/rpET21 was grown at 37°C with agitation at 250 rpm in LB broth (peptone 10g/L, yeast extract 5g/L, NaCl 10g/L) containing 100 µg/ml of carbenicillin (Sigma-Aldrich Canada Ltd., Oakville, Canada), until the A₆₀₀ reached a value of 0.5. In order to induce the production of nontypeable H. influenzae BVH-NTHI1-His●Tag recombinant polypeptide, the cells were incubated for 3 additional hours at 30°C in presence of IPTG at a final concentration of 1 mM. Induced cells from a 500 ml culture were pelleted by centrifugation and frozen at -70°C.

The purification of the recombinant polypeptides from the soluble cytoplasmic fraction of IPTG-induced Tuner(DE3) /rpET21 was done by affinity chromatography based on the properties of the His●Tag sequence (6 consecutive histidine residues) to bind to divalent cations (Ni²⁺) immobilized on the His●Bind metal chelation resin. Briefly, the pelleted cells obtained from a 500 mL IPTG-induced culture was resuspended in lysis buffer (20 mM Tris, 500 mM NaCl, 5 mM imidazole, pH 7.9) containing 1 mM of phenylmethylsulfonyl fluoride (PMSF; Sigma), sonicated and centrifugated at 16,000 X g for 30 min to remove debris. The supernatant was deposited on a Ni-NTA agarose column (QIAgen, Mississauga, Ontario, Canada). The nontypeable H. influenzae BVH-NTHI1-His●Tag recombinant polypeptide was eluted with 250 mM imidazole-500mM NaCl-20 mM Tris pH 7.9. Removal of salt and imidazole from the sample was performed by dialysis against PBS at 4°C. The quantity of recombinant polypeptide obtained from the soluble fraction of E. coli was estimated by MicroBCA (Pierce, Rockford, Illinois).

### EXAMPLE 4

This example illustrates the analysis of the immunogenicity and the protective ability of mice against nontypeable H. influenzae infection following immunization with BVH-NTHI1.

Groups of 5 female BALB/c mice (Charles River, Ontario, Canada) were immunized intranasally three times at two-week intervals with 25 µg of affinity purified BVH-NTHI1-His●Tag recombinant polypeptide in presence of the E. coli heat-labile toxin adjuvant (LT; 1 µg; Cedarlane Laboratories Ltd, Hornby, Canada) or, as a control, with adjuvant alone in PBS. Blood samples were collected from the orbital sinus on the day prior to each immunization and 14 days following the third (day 42) injection. Antibody titers were determined by ELISA on heat-killed and outer membrane vesicules of the nontypeable H. influenzae strain 12085. The secondary antibody used was a goat anti-mouse IgG + IgM (H+L) (Fc specific) conjugated to alcaline phosphatase (Jackson Immunoresearch Labs, Mississauga, Ontario). The reactive titer of an antiserum was defined as the reciprocal of the dilution showing a two-fold increase in absorbance over that obtained with the pre-immune serum sample. To investigate whether immune responses elicited by BVH-NTHI1 were functionally significant, in vivo protective efficacy was evaluated 14 days later in mice challenged intrapulmonarily with approximately 2 x 10⁵ CFU of the nontypeable H. influenzae strain 12085. Samples of the nontypeable H. influenzae challenge inoculum were plated on chocolate agar plates to verify the challenge dose. To measure the effectiveness of vaccination in limiting in vivo growth of nontypeable H. influenzae, mice were killed by an intraperitoneal injection of sodium pentobarbital (Euthanyl^{™}) 5 h after infection. Bronchoalveolar lavages were assessed for bacterial clearance by plating of serial dilutions for bacterial count determination. Mice injected with adjuvant only were used as negative controls.

Results of the immunogenicity study (Table 2) showed that the level of specific antibodies in serum of immunized animals rose by around 1000-fold relative to control serum. The mean titer measured on OMP and heat-killed samples diverged by only one dilution, confirming that the BVH-NTHI1 polypeptide really represents a surface-exposed antigen.

**Table 2. Effect of systemic immunization on NTHI-directed antibody levels in serum^{a}**

| NTHI 12085 | ELISA titer | |
|---|---|---|
| | Control | Immune |
| Outer membrane polypeptides | 200 | 184 320 |
| Heat-killed | 360 | 368 640 |

| | | |
|---|---|---|
| a) Serum samples were collected from immune and control mice on day 42 of the immunization regimen. | | |

Results of the protection study (Table 3) showed that strain 12085 readily multiplied in the lungs of the control animals such that by 5 hours postchallenge, the number of viable nontypeable H. influenzae cells had increased by 200%. In contrast, substantial pulmonary clearance of strain 12085 by the BVH-NTHI1-immunized mice was clearly evident, whereas only 35% of the original number of organisms deposit in the lungs of the immune animals were recovered by 5-hour postchallenge.

**Table 3. Effect of systemic immunization on pulmomary clearance of nontypeable H. influenzae 12085^{a}.**

| BALB/c Mice | Nontypeable H. influenzae 12085 (x 10⁴) CFU | |
|---|---|---|
| | 0 h | 5 h |
| Control | 4,6 | 9,2 |
| Immunized | nd | 3.2^{b} |

| | | |
|---|---|---|
| a) Each value represents a mean of five animals at each time. b) **P < 0.0098 calculated according to the unpaired t test with Welch's correction and compared to control. | | |

### EXAMPLE 5

This example illustrates the recognition of recombinant polypeptides from nontypeable H. influenzae strain 12085 by human sera and by antisera from protected mice.

A standard Western blot technique was used to investigate whether purified recombinant polypeptides were recognized by human sera. In addition, antisera from mice immunized with outer membrane proteins preparations were tested against the purified recombinant polypeptides. Mice immunized with these preparations demonstrated increased pulmonary clearance following a challenge with nontypeable H. influenzae strain 12085. For the preparation of antigens, purified recombinant polypeptides were treated with sample buffer containing SDS and 2-mercaptoethanol for 5 min at 100°C. Polypeptides were resolved by SDS-PAGE according to the method of Laemmli, U.K. (1970) Cleavage of structural proteins during the assembly of the head of the bacteriophage T4. Nature, 227 :680-685. After SDS-PAGE, the polypeptides were transferred electrophoretically from the gel to nitrocellulose paper by the method (Towbin, H., Staehelin, T. and Gordon, J. (1979) Electrophoretic transfer of protein from polyacrylamide gels to nitrocellulose sheets : procedure and some applications. Proc. Natl. Acad. Sci. USA, 76 :4350-4354) and probed with human or mouse antisera. The detection of antigens reactive with the antibodies was performed by indirect enzyme-immunoassay using conjugated anti-mouse or anti-human immunoglobulins and a color substrate. Results in Table 4 show that most polypeptides have already been seen by the human immune system. As vaccine candidates, these polypeptides thus have a potential to induce a strong immune response in humans. Moreover, most polypeptides were recognized by sera from protected mice. The serum reactivities correlate to increased pulmonary clearance in the mouse model of infection.

**Table 4. Reactivities of normal human sera and antisera from protected mice with nontypeable H. influenzae 12085 recombinant polypeptides.**

| Seq. ID. | Polypeptide | Serum reactivity^{a} | | Pulmonary clearance (%)^{b} |
|---|---|---|---|---|
| | | Human | Mouse | |
| 2 | BVH-NTHI1 | ++ | +++ | 65 |
| 4 | BVH-NTHI2 | + | +++ | 36 |
| 6 | BVH-NTHI3 | + | ++ | 56 |
| 8 | BVH-NTHI4 | ++ | +++ | 98 |
| 10 | BVH-NTHI5 | - | ++ | nd^{c} |
| 16 | BVH-NTHI8 | - | + | nd |
| 18 | BVH-NTHI9 | ++ | ++ | nd |
| 20 | BVH-NTHI10 | +++ | +++ | 44 |
| 24 | BVH-NTHI12 | ++++ | - | 20 |

| | | | | |
|---|---|---|---|---|
| a) The number of +'s indicates the reaction intensity The «-» sign indicates No reaction b) Mice were immunised with the respective recombinant polypeptides and pulmonary clearance was measured as in Example 4 c) nd, not done | | | | |

## Claims

1. An isolated polypeptide selected from:
(a) a polypeptide having greater than 99% identity to the amino acid sequence set forth in SEQ ID NO:10;
(b) a polypeptide comprising the amino acid sequence set forth in SEQ ID NO: 10;
(c) the polypeptide of (a) or (b), wherein the N-terminal Met residue is deleted; and
(d) the polypeptide of (a) or (b), wherein the secretory amino acid sequence is deleted;
wherein the isolated polypeptide can elicit an immune response to *Haemophilus influenzae.*

2. An immunogenic pharmaceutical composition comprising an isolated polypeptide selected from:
(a) a polypeptide having at least 70% identity to the amino acid sequence set forth in SEQ ID NO:10;
(b) a polypeptide having at least 80% identity to the amino acid sequence set forth in SEQ ID NO:10;
(c) a polypeptide having at least 85% identity to the amino acid sequence set forth in SEQ ID NO:10;
(d) a polypeptide having at least 90% identity to the amino acid sequence set forth in SEQ ID NO:10;
(e) a polypeptide having at least 95% identity to the amino acid sequence set forth in SEQ ID NO:10;
(f) a polypeptide comprising the amino acid sequence set forth in SEQ ID NO: 10;
(g) a polypeptide that comprises an immunogenic fragment consisting of at least 15 contiguous amino acids from the amino acid sequence set forth in SEQ ID NO: 1 0, wherein the immunogenic fragment can elicit an immune response to *Haemophilus influenzae*;
(h) the polypeptide of (a), (b), (c), (d), (e), (f) or (g), wherein the N-terminal Met residue is deleted; and
(i) the polypeptide of (a), (b), (c), (d), (e), (f) or (g), wherein the secretory amino acid sequence is deleted;
wherein the isolated polypeptide can elicit an immune response to *Haemophilus influenzae*;
and a pharmaceutically acceptable carrier, diluent or adjuvant.

3. A pharmaceutical composition according to claim 2 wherein the polypeptide is as defined in claim 1.

4. A pharmaceutical composition according to claim 2 or 3 for use in the prophylactic or therapeutic treatment of otitis media, sinusitis, bronchitis, pneumonia and meningitis or bacteremia mediated by *Haemophilus influenzae* bacterial infection.

5. A pharmaceutical composition according to claim 2 or 3 for use in the prophylactic or therapeutic treatment of *Haemophilus influenzae* bacterial infection in an individual susceptible to *Haemophilus influenzae* infection.

6. A pharmaceutical composition according to claim 2 or 3 for use in the prophylactic or therapeutic treatment of *Haemophilus influenzae* infection.

7. A pharmaceutical composition according to any one of claims 2 to 6 wherein (a) *Haemophilus influenzae* is Nontypeable *Haemophilus influenzae* or (b) *Haemophilus influenzae* is Typeable *Haemophilus influenzae*.

## Patentansprüche

1. Isoliertes Polypeptid ausgewählt aus:
(a) einem Polypeptid mit mehr als 99 % Identität mit der in SEQ ID NO: 10 dargestellten Aminosäuresequenz;
(b) einem Polypeptid, das die in SEQ ID NO: 10 dargestellte Aminosäuresequenz umfasst;
(c) dem Polypeptid von (a) oder (b), wobei der N-terminale Met-Rest deletiert ist; und
(d) dem Polypeptid von (a) oder (b), wobei die sekretorische Aminosäuresequenz deletiert ist;
wobei das isolierte Polypeptid eine Immunantwort gegen Haemophilus influenzae auslösen kann.

2. Immunogene pharmazeutische Zusammensetzung, umfassend ein isoliertes Polypeptid ausgewählt aus:
(a) einem Polypeptid mit mindestens 70 % Identität mit der in SEQ ID NO: 10 dargestellten Aminosäuresequenz;
(b) einem Polypeptid mit mindestens 80 % Identität mit der in SEQ ID NO: 10 dargestellten Aminosäuresequenz;
(c) einem Polypeptid mit mindestens 85 % Identität mit der in SEQ ID NO: 10 dargestellten Aminosäuresequenz;
(d) einem Polypeptid mit mindestens 90 % Identität mit der in SEQ ID NO: 10 dargestellten Aminosäuresequenz;
(e) einem Polypeptid mit mindestens 95 % Identität mit der in SEQ ID NO: 10 dargestellten Aminosäuresequenz;
(f) einem Polypeptid, das die in SEQ ID NO: 10 dargestellte Aminosäuresequenz umfasst;
(g) einem Polypeptid, das ein immunogenes Fragment umfasst, bestehend aus mindestens 15 zusammenhängenden Aminosäuren aus der in SEQ ID NO: 10 dargestellten Aminosäuresequenz, wobei das immunogene Fragment eine Immunantwort gegen Haemophilus influenzae auslösen kann;
(h) dem Polypeptid von (a), (b), (c), (d), (e), (f) oder (g), wobei der N-terminale Met-Rest deletiert ist; und
(i) dem Polypeptid von (a), (b), (c), (d), (e), (f) oder (g), wobei die sekretorische Aminosäuresequenz deletiert ist;
wobei das isolierte Polypeptid eine Immunantwort gegen Haemophilus influenzae auslösen kann;
und ein(en) pharmazeutisch verträglichen/verträgliches Träger, Verdünnungsmittel oder Adjuvans.

3. Pharmazeutische Zusammensetzung gemäß Anspruch 2, wobei das Polypeptid so ist, wie in Anspruch 1 definiert.

4. Pharmazeutische Zusammensetzung gemäß Anspruch 2 oder 3 zur Verwendung in der prophylaktischen oder therapeutischen Behandlung von Otitis Media, Sinusitis, Bronchitis, Lungenentzündung und Meningitis oder Bakteriämie, vermittelt durch Haemophilus influenzae-Bakterieninfektion.

5. Pharmazeutische Zusammensetzung gemäß Anspruch 2 oder 3 zur Verwendung in der prophylaktischen oder therapeutischen Behandlung von Haemophilus influenzae-Bakterieninfektion in einem Individuum, das für Haemophilus influenzae-Infektion anfällig ist.

6. Pharmazeutische Zusammensetzung gemäß Anspruch 2 oder 3 zur Verwendung in der prophylaktischen oder therapeutischen Behandlung von Haemophilus influenzae-Infektion.

7. Pharmazeutische Zusammensetzung gemäß mindestens einem der Ansprüche 2 bis 6, wobei (a) Haemophilus influenzae nicht-typisierbarer Haemophilus influenzae oder (b) Haemophilus influenzae typisierbarer Haemophilus influenzae ist.

## Revendications

1. Polypeptide isolé choisi parme :
(a) un polypeptide ayant plus de 99 % d'identité avec la séquence d'acides aminés exposée dans SEQ ID NO : 10 ;
(b) un polypeptide comprenant la séquence d'acides aminés exposée dans SEQ ID NO : 10 ;
(c) le polypeptide de (a) ou (b), dans lequel le résidu Met N-terminal est délété ; et
(d) le polypeptide de (a) ou (b), dans lequel la séquence d'acides aminés sécrétoire est délétée ;
dans lequel le polypeptide isolé peut susciter une réponse immunitaire à *Haemophilus influenzae.*

2. Composition pharmaceutique immunogène comprenant un polypeptide isolé choisi parme :
(a) un polypeptide ayant au moins 70 % d'identité avec la séquence d'acides aminés exposée dans SEQ ID NO : 10 ;
(b) un polypeptide ayant au moins 80 % d'identité avec la séquence d'acides aminés exposée dans SEQ ID NO : 10 ;
(c) un polypeptide ayant au moins 85 % d'identité avec la séquence d'acides aminés exposée dans SEQ ID NO : 10 ;
(d) un polypeptide ayant au moins 90 % d'identité avec la séquence d'acides aminés exposée dans SEQ ID NO : 10 ;
(e) un polypeptide ayant au moins 95 % d'identité avec la séquence d'acides aminés exposée dans SEQ ID NO : 10 ;
(f) un polypeptide comprenant la séquence d'acides aminés exposée dans SEQ ID NO : 10 ;
(g) un polypeptide qui comprend un fragment immunogène consistant en au moins 15 acides aminés contigus de la séquence d'acides aminés exposée dans SEQ ID NO : 10, dans lequel le fragment immunogène peut susciter une réponse immunitaire à *Haemophilus influenzae ;*
(h) le polypeptide de (a), (b), (c), (d), (e), (f) ou (g), dans lequel le résidu Met N-terminal est délété ; et
(i) le polypeptide de (a), (b), (c), (d), (e), (f) ou (g), dans lequel la séquence d'acides aminés sécrétoire est délétée ;
dans laquelle le polypeptide isolé peut susciter une réponse immunitaire à *Haemophilus influenzae ;*
et un vecteur, diluant ou adjuvant pharmaceutiquement acceptable.

3. Composition pharmaceutique selon la revendication 2, dans laquelle le polypeptide est tel que défini dans la revendication 1.

4. Composition pharmaceutique selon la revendication 2 ou 3, pour une utilisation dans le traitement prophylactique ou thérapeutique de l'otite moyenne, la sinusite, la bronchite, la pneumonie et la méningite ou une bactériémie médiée par une infection bactérienne à *Haemophilus influenzae.*

5. Composition pharmaceutique selon la revendication 2 ou 3, pour une utilisation dans le traitement prophylactique ou thérapeutique d'une infection bactérienne à *Haemophilus influenzae* chez un individu sensible à une infection à *Haemophilus influenzae.*

6. Composition pharmaceutique selon la revendication 2 ou 3, pour une utilisation dans le traitement prophylactique ou thérapeutique d'une infection à *Haemophilus influenzae.*

7. Composition pharmaceutique selon l'une quelconque des revendications 2 à 6, dans laquelle (a) *l'Haemophilus influenzae* est un *Haemophilus influenzae* ne pouvant pas être typé ou (b) *l'Haemophilus influenzae* est un *Haemophilus influenzae* pouvant être typé.
